(19) Europäisches Patentamt European Patent Office Office européen des brevets

(11) **EP 4 460 830 B1**

(12) **EUROPEAN PATENT SPECIFICATION**

(45) Date of publication and mention of the grant of the patent:
**15.04.2026 Bulletin 2026/16**

(21) Application number: **23700316.5**

(22) Date of filing: **06.01.2023**

(51) International Patent Classification (IPC):
*G16B 30/10* (2019.01)      *G16B 40/20* (2019.01)

(52) Cooperative Patent Classification (CPC):
**G16B 30/10; G16B 40/20;** G16B 10/00; Y02A 90/10

(86) International application number:
**PCT/GB2023/050022**

(87) International publication number:
**WO 2023/131795 (13.07.2023 Gazette 2023/28)**

(54) **METHOD AND APPARATUS FOR DETECTING PATHOGENS**

VERFAHREN UND VORRICHTUNG ZUM NACHWEIS VON PATHOGENEN

PROCÉDÉ ET APPAREIL DE DÉTECTION D'AGENTS PATHOGÈNES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.01.2022 GB 202200151**

(43) Date of publication of application:
**13.11.2024 Bulletin 2024/46**

(73) Proprietors:
- **Earlham Enterprises Ltd**
  **Colney Norwich NR4 7UZ (GB)**
- **The Natural History Museum Trading Company Limited**
  **London SW7 5BD (GB)**

(72) Inventors:
- **CLARK, Matthew**
  **London SW7 5BD (GB)**
- **MISRA, Raju**
  **London SW7 5BD (GB)**
- **LEGGETT, Richard**
  **Norwich NR4 7UZ (GB)**
- **ALSTON, Mark**
  **Norwich NR4 7UZ (GB)**
- **MARTIN, Samuel**
  **Norwich NR4 7UZ (GB)**

(74) Representative: **Appleyard Lees IP LLP**
**G Mill**
**Dean Clough Industrial Park**
**Halifax HX3 5AH (GB)**

(56) References cited:
**CN-A- 109 686 408      US-A1- 2015 159 201**
**US-A1- 2018 365 375**

- **DILTHEY ALEXANDER T. ET AL: "Strain-level metagenomic assignment and compositional estimation for long reads with MetaMaps", NATURE COMMUNICATIONS, vol. 10, no. 1, 11 July 2019 (2019-07-11), XP093036031, Retrieved from the Internet <URL:https://www.nature.com/articles/s41467-019-10934-2.pdf> [retrieved on 20230329], DOI: 10.1038/s41467-019-10934-2**
- **LI PO-E ET AL: "PanGIA: A Metagenomics Analytical Framework for Routine Biosurveillance and Clinical Pathogen Detection", BIORXIV, 23 April 2020 (2020-04-23), XP093036021, Retrieved from the Internet <URL:https://www.biorxiv.org/content/10.1101/2020.04.20.051813v1.full.pdf> [retrieved on 20230330], DOI: 10.1101/2020.04.20.051813**

**Description**

**Field**

We describe a computer implemented method for detecting pathogens within a sample and a system for implementing the method, particularly using metagenomic analysis.

**Background**

Various methods for characterising microbiomes are known in the art, including many which use metagenomic analysis. US2021/222231 describes a method for monitoring and managing the microbiome of a facility and taking corrective action to prevent harm to the occupants. US 2018/365375 A1 discloses a method for metagenomic read classification and pathogen detection based on a two-step approach of a first sequence comparison to a general database of pathogens followed by a second comparison to sequences that are selected based on a preliminary assignment from the first comparison. However, no incremental confidence score based on both comparisons is disclosed. US 2018/365375 A1 also discloses including coverage of a reference genome in the assessment, but does not calculating a genome spread score by dividing the reference genome into segments and accounting for how many reads map to each segment.

"Optimized DNA extraction and metagenomic sequencing of airborne microbial communities" by Jiang et al, published in Nature Protocols 10, 768-799 (2015) describes an optimized protocol for extracting up to tens of nanograms of airborne microbial genomic DNA from collected particulate matter. As explained in the paper, for the purpose of identifying microbial species, HiSeq reads are aligned to a cohort of nonredundant National Center for Biotechnology Information (NCBI) complete genomes using the Short Oligonucleotide Analysis Package (SOAP) alignment tool, which is typically faster to run than the Basic Local Alignment Search Tool (BLAST) or the BLAST-like Alignment Tool (BLAT).

"Longitudinal survey of microbiome associated with particular matter in a megacity" by Qin et al published in Genome Biology 21, Article number: 55 (2020) describes a longitudinal metagenomic survey of airborne samples in Beijing. "Characterization of the public transit air microbiome and resistome reveals geographical specificity" by Leung et al published in Microbiome 9, Article number: 112 (2021) describes a study of public transit air microbiomes in six cities. Both of these papers describe the use of MetaPhlAn2 which works by identifying specific marker genes.

The present applicant has recognised the need for a more accurate real-time method for determining whether a pathogen is present in a sample, for example an air sample. There needs to be high sensitivity for pathogen and an extremely low false positive rate to avoid triggering unnecessary alerts.

SUMMARY OF THE INVENTION

The invention is set out in the appended claims.

SUMMARY OF RELATED DISCLOSURE

According to the present invention there is provided an apparatus and method as set forth in the appended claims. Other features of the invention will be apparent from the dependent claims, and the description which follows.

We describe a real-time method for detecting a pathogen in a sample, the method comprising: inputting a plurality of sequence reads which are obtained from the sample using nucleic acid sequencing. For each input sequence read in the plurality of sequence reads, the method comprises comparing, using a first classification algorithm, the input sequence read to a plurality of target sequences in a first database, wherein the first database comprises genomic sequences for pathogens; identifying, using the first classification algorithm, a set of hits for the input sequence read, wherein each hit is a target sequence which matches the input sequence read; assigning, using the set of hits, the input sequence read to a taxon; and classifying the input sequence read as potentially pathogenic when the assigned taxon corresponds to a pathogen taxon. For each input sequence read classified as potentially pathogenic, the method further comprises: comparing, using a second classification algorithm, the potentially pathogenic input sequence read to a plurality of target sequences in a second database, wherein the second database is selected based on the corresponding pathogen taxon; and confirming the classification of the potentially pathogenic input sequence read as pathogenic when the potentially pathogenic input sequence read matches one or more target sequences in the second database. The method further comprises calculating a confidence score for each input sequence read incrementing the confidence score by a first fixed amount when the input sequence read is classified as potentially pathogenic and by incrementing the confidence score by a second fixed amount when each input sequence read which is classified as potentially pathogenic is confirmed to be pathogenic, and comparing the confidence score to a confidence score threshold to determine whether each input sequence read is a match to a pathogen.

The method thus uses two different classification algorithms whereby a more accurate detection rate may be obtained. The method may be considered to be a two-stage classification process with the second classification stage being used to give greater confidence in the first stage classification when the two match. By contrast, known methods typically use only a single classification stage. The second classification algorithm may be more accurate or more specific than the first classification algorithm. Accuracy (or specificity) may be measured by using the lower false positive rate, i.e. the percentage of the closely related species that are falsely classified as pathogens. A more accurate algorithm may have a false positive rate close to 0% and a less accurate algorithm may have a false positive rate which is higher than the more accurate algorithm and which may be up to 6%.

Ensuring that there is a low overall false positive rate (i.e. a false positive rate close to 0%) may be particularly important when testing for pathogens in an environmental rather than clinical setting. Detecting a pathogen may result in an alert being triggered and the impact of a false alert may be particularly damaging, for example, if an evacuation is initiated but not needed. Accurately detecting a pathogen within an environmental sample is further complicated because there are typically very low numbers of sequence reads for the pathogen (e.g. perhaps as few as 5 pathogen reads per 50,000 reads). An accurate detection, even with such low levels, is important. Typically, a more accurate classification algorithm runs more slowly than a less accurate algorithm because a more accurate classification algorithm typically has higher computational complexity. Nevertheless, the method may still be performed in real-time because the second classification algorithm is only used once a possible pathogen is identified by the first classification algorithm and by using a second database which is selected based on the genus of the possible pathogen.

The first classification algorithm may be a seed and extend local alignment search tool, e.g. BLAST or a k-mer matching tool, e.g. Kraken. BLAST is described for example in "Basic Local Alignment Search Tool" by Altschul et al published in Journal of Molecule Biology 1009, Oct 5; 215(3):403-10. Kraken is described for example in "Improved metagenomic analysis with Kraken 2" by Wood et al in Genome Biol, 2019; 20(1): 257.

The pathogen may be a bacterial, fungal or viral pathogen. As set out above, the first database comprises genomic sequences for pathogens. The first database may only contain nucleic acid sequences which have a taxonomy identification as a pathogen, e.g. a bacterial, fungal or a viral pathogen. Alternatively, the first database may comprise sequences from all domains (e.g. plant, animal, bacteria and virus sequences) or selected domains (e.g. bacteria and virus sequences) and may thus comprise sequences for pathogens and non-pathogens. The first database may be generated using a standard nucleotide database, e.g. the nucleotide (nt) database published by the National Center for Biotechnology Information (NCBI), which is filtered according to taxonomy identification. The first database may be limited to bacterial, fungal and/or viral sequences (i.e. both pathogens and non-pathogens) whereby the step of identifying a set of hits should take less time than if the first database contained all sequences (e.g. including plant and animal sequences). However, it may be possible for the first database to contain all sequences if the first classification algorithm is able to identify a set of hits in real-time. The first database may be termed a nucleotide database.

The second classification algorithm may be a strain-level metagenomic assignment and compositional estimation algorithm, known as MetaMaps. The second database may be a genus-level database, i.e. a database of sequences for a particular pathogen and its relatives. The second database may be generated using a standard database, e.g. RefSeq, by identifying each genomic sequence in the standard database from a particular genus, and selecting each identified genome to be in the second database. The RefSeq database is described for example in "Reference sequence (RefSeq) database at NCBI: current status, taxonomic expansion and functional annotation" by O'Leary et al published in Nucleic Acids Res 2016 Jan 4; 44(D1): D733-45.

The second database may be based on the corresponding pathogen taxon by selecting one of a genus-level database, a family level database, a species-level database or a subspecies level database as the second database. For example, a genus-level database may be created by calculating a score relative to a particular pathogen for each reference sequence within a genus and discarding at least some of the reference sequences which do not meet a score criteria, e.g. discarding references with a score below a threshold score. All reference sequences which meet the score criteria may be retained. The score may be any suitable score, e.g. average nucleotide identity (ANI) score when compared to a particular pathogen. The threshold score may be any suitable score value for example specified as X%. Merely as examples, suitable values for X are 87 or 95 depending on the desired size of the database. The genus-level database may comprise only a fixed proportion of the reference sequences which do not meet the score criteria. The fixed proportion may be 50% (i.e. 1 in 2), 20% (i.e. 1 in 5), 10% (i.e. 1 in 10) or 5% (i.e. 1 in 20). In other words, all reference sequences within X% of the pathogen may be retained together with at least some of the reference sequences which are below X%.

Identifying a set of hits for the input sequence read may comprise identifying a query sequence as a hit when the query sequence meets a minimum matching threshold which may be configurable by a user. The number of hits within each set may be defined by identifying a highest scoring hit within the set and selecting all hits having a score within a score threshold (e.g. 90%) of the highest score. The number within the set may be limited to a maximum number (e.g. 20 hits). The score threshold and the maximum number may be configurable by the user. In other words, the set of hits may comprise only high-quality matches.

Assigning the input sequence read to a taxon may be done using a lowest common ancestor algorithm on the set of hits. For example, taxonomic paths for each hit within the set of hits may be compared, and selecting the lowest common ancestor within each compared taxonomic path as the taxon. The lowest common ancestor may be last node within each taxonomic path which is the same. For example, if a read returned hits to Escherichia coli O25 and Escherichia coli PCN061 (two strains of E. coli), the Lowest Common Ancestor would be Escherichia coli. However, if a read returned hits to "Escherichia coli" (species) and "Klebsiella pneumoniae" (species), the Lowest Common Ancestor would be Enterobacteriaceae (family). In other words, an input sequence is assigned to a single taxon which may be a species or a higher level (e.g. genus) depending on the set of hits. Once assigned to a taxon, an input sequence read is classified as a pathogen when the assigned taxon corresponds to a pathogen taxon. The assigned taxon may be determined to correspond to a pathogen taxon by comparing the assigned taxon to a list of pathogen taxa. When the assigned taxon matches a taxon in the list of pathogen taxa, the assigned taxon may be determined to correspond. When the assigned taxon does not match a taxon in the list of pathogen taxa, the sequence read may be classified as a non-pathogen.

In addition to matching the assigned taxon to the list, various criteria may be required to be met before the input sequence read is classified as pathogenic. The criteria may comprise one or more of an identity value exceeding an identity threshold, a query cover value exceeding a query cover threshold, a combined score based on the identity value and the query cover value exceeding a combined score threshold and the input sequence read having a minimum length. The identity value may be the amount (e.g. percentage) of the input sequence read which matches a target sequence and an example identity threshold is 70%. The query cover value may be the amount (e.g. percentage) of the overall length of the input sequence read which matches a target sequence and an example query cover threshold is 40%. The minimum length may be defined as 100 base pairs.

The method may further comprise determining whether the input sequence read is associated with a virulence factor and incrementing the confidence score by a third fixed amount when the input sequence is determined to be virulent. Determining whether the input sequence read is associated with a virulence factor may comprise comparing the input sequence read against virulence factor database using the first classification algorithm (e.g. BLAST). The third fixed amount may be smaller than the first and second fixed amounts. In this way, the classification as a pathogen by the first and second classification algorithm is more important (or more heavily weighted) than the determination that the input sequence read is associated with a virulence factor. This reflects the fact that non-pathogenic viruses and bacteria may also have a virulence factor. The first fixed amount may be equal to the second fixed amount to reflect the equal weighting applied to both the first and second classification algorithms.

The method above describes a method for determining whether each input sequence read is a match to a bacterial or viral pathogen. An alert may be output when there are more than a threshold number of input sequence reads which are determined to be a match to a bacterial or viral pathogen. The threshold number may be one or may be higher, e.g. three, to increase sensitivity and reduce the risk of a sample falsely being identified as containing a pathogen.

When a plurality of input sequence reads is determined to be a match to a bacterial or viral pathogen and thus have been classified as pathogenic based on the confidence score, the method may further comprise additional analysis to further reduce the risk of a false positive. For example, for each pathogen which is identified, the method may comprise determining the number of input sequence reads which are classified as pathogenic for the particular pathogen; and calculating a pathogen confidence score associated with the particular pathogen from each confidence score for an input sequence read for the particular pathogen. The pathogen confidence score may be the mean of all the confidence scores for each input sequence read which is classified as pathogenic for the particular pathogen. The pathogen confidence score may be compared to a pathogen confidence score threshold to confirm whether the pathogen has been correctly identified.

We also describe another method which may be combined or used separately from the method above when there are plurality of input sequence reads classified as pathogenic. For each pathogen which is identified, the method may comprise determining the number of input sequence reads which are classified as pathogenic for the particular pathogen, determining the number of input sequence reads $n_g$ which are classified in the same genus as the particular pathogen and calculating a pathogen to relative ratio using the numbers of input sequence reads determined in the previous steps. The pathogen-to-relative ratio $R_p$ may be calculated as

$$R_p = \frac{n_p}{m_g(n_g - n_p) + n_p}$$

where $n_p$ is the number of pathogen reads, $n_g$ is the number of reads classified to the same genus as the pathogen and $m_g$ is a per-genus multiplier. The per-genus multiplier may be set to 1 as a default value. The per-genus multiplier may have a value below 1 when there is known to be a large background of the same genus to the particular pathogen. The pathogen-to-relative ratio may be compared to a pathogen-to-relative threshold to confirm whether the pathogen has been correctly identified.

We also describe another method which may be combined or used separately from the methods above when there are plurality of input sequence reads determined to be a match to a bacterial or viral pathogen. For each pathogen which is identified, the method may comprise dividing the identified pathogen into a plurality of segments, counting the number of input sequence reads in each segment which have been classified as pathogenic, and calculating, using the counted number of input sequence reads in each segment, a genome spread value which is indicative of a distribution of the pathogenic input sequence reads across the pathogen. The genome spread $g_s$ may then be calculated as

$$g_s = 1 - V_d$$

where $V_d$ is a total variation distance which is calculated by comparing a distribution of the counted number of input sequence reads in each segment to a uniform distribution across the pathogen of input sequence reads which have been classified as pathogenic. The uniform distribution may be determined using the mean number of pathogenic input sequence reads per segment. The genome spread $g_s$ may be a probability distribution with a number close to one representing a uniform distribution and a number close to zero indicating that the distribution is nothing like a uniform distribution. The genome spread value may be compared to a genome spread threshold to confirm whether the pathogen has been correctly identified. When the genome spread is below the genome spread threshold, the reads are likely to be clustered in segments and thus there is more likely to be a misclassification of each read as containing a pathogen.

We also describe another method which may be combined or used separately from the methods above when there are plurality of input sequence reads classified as pathogenic. For each pathogen which is identified, the method may further comprise determining the number of input sequence reads $n_u$ which are classified as pathogenic for a particular pathogen and which are unique to that particular pathogen; determining the number of input sequence reads $n_n$ which are classified as pathogenic for the particular pathogen and which are not unique to that particular pathogen; and calculating a uniqueness ratio using the numbers of input sequence reads determined in the previous steps. The uniqueness ratio may be calculated as:

$$U_p = \frac{n_u}{n_n}$$

where $n_u$ is the number of unique pathogenic reads and $n_n$ is the number of non-unique pathogenic reads. The uniqueness ratio may be compared to a uniqueness threshold to confirm whether the pathogen has been correctly identified. The uniqueness ratio may also be incorporated into the confidence score calculation, e.g. in a weighted sum.

As an alternative, or in addition to using the uniqueness ratio, when an input sequence read is identified as being unique, the confidence score for that individual read may be incremented by a fourth fixed amount. The fourth fixed amount may be larger than any one of the first, second and third fixed amounts to reflect the importance of matching a sequence within a pathogen that is unique to that pathogen. An input sequence read may be identified as being unique by comparing each input sequence read to a database containing only target sequences within a genome of a pathogen which are unique to that pathogen.

Some or all of the methods described above may be combined to generate an overall score for the particular pathogen using a weighted sum of some or all of the pathogen confidence score, the pathogen to relative ratio, the genome spread value and the uniqueness ratio. At least one of the number of input sequence reads $n_p$ which are determined to be a match to a particular pathogen and the pathogen confidence score may be used as a multiplier in the weighted sum. The overall score for the particular pathogen may be calculated using

$$S_P = n_p \, s_{mean} \, (1 + \alpha R_p + \beta s_g + \delta U_p)$$

where $n_p$ is the number of input sequence reads which are determined to be a match to a particular pathogen, $s_{mean}$ is the pathogen confidence score, $R_p$ Is the pathogen-to-relative ratio, $s_g$ is the genome spread value, $U_p$ is the uniqueness ratio and $\alpha$, $\beta$, $\delta$ are constants (e.g. 1 initially and configurable to change the importance of each term).

Once the score for a particular pathogen has been calculated, the method may further comprise determining whether there is another pathogen which has been identified. The methods described above may then be repeated for each additional pathogen which is identified. An overall score for the sample may be obtained by summing all the pathogen scores, i.e.

$$S_S = \sum_{i=1}^{q} S_p$$

where $S_P$ is the score for a particular pathogen, q is the number of pathogens identified in the sample and $S_S$ is the overall score for the sample.

The sample may be obtained from the environment, for example air, water or soil. Alternatively, the sample may be a clinical sample. The sample may be obtained using any suitable method or device. The plurality of sequence reads are obtained using nucleic acid sequencing, for example whole genome shotgun sequencing which is a standard technique for randomly sampling from a genome/genomes. There are typically very low numbers of sequence reads for the pathogen (e.g. perhaps as few as 5 pathogen reads per 50,000 reads) in the environment. The target sensitivity for the method may be 0.1 parts per litre (PPL). The number of reads is dependent on the background level of bacteria in the environment (as explained for example in the textbook "Microbial Ecology" by Bowers et al published in 2011). As noted in the table below, there may be as few as 5 pathogen reads in the whole sample and thus it is important to be sure that a single read is reliably identified.

| PPL (a) | Background level (b) | Bacteria per litre (c) = ((b)/100) | Pathogen DNA proportion (d) = ((a)/(b)) | Pathogen reads per 50,000 reads (d) *50,000 |
|---|---|---|---|---|
| 0.1 | Low (10,000 bacteria /m$^2$) | 10 | 0.01 | 500 |
| 0.1 | High (1,000,000 bacteria /m$^2$) | 1,000 | 0.0001 | 5 |

We also describe a non-transitory data carrier carrying code which, when implemented on a processor, causes the processor to carry out any of the methods described herein. We also describe processor control code to implement the above-described methods, for example on a general purpose computer system or on a digital signal processor (DSP). The code may be provided on a carrier such as a disk, a microprocessor, CD- or DVD-ROM, programmed memory such as non-volatile memory (e.g. Flash) or read-only memory (firmware), or on a data carrier such as an optical or electrical signal carrier. Code (and/or data) to implement the method described herein may comprise source, object or executable code in a conventional programming language (interpreted or compiled) such as Python, C, or assembly code, code for setting up or controlling an ASIC (Application Specific Integrated Circuit) or FPGA (Field Programmable Gate Array), or code for a hardware description language such as Verilog (RTM) or VHDL (Very high speed integrated circuit Hardware Description Language). As the skilled person will appreciate, such code and/or data may be distributed between a plurality of coupled components in communication with one another.

We also describe a system, e.g. a computerised system comprising one or more processors, e.g. microprocessors, working memory and program memory coupled to one or more of the components of the system. The one or more processors may be configured to execute instructions in said memory to cause the system to carry out the method described above. The system may also comprise one or more devices configured to capture a sample and perform nucleic acid sequencing on the captured sample. The one or more devices may comprise a device for extracting nucleic acid and optionally a device for amplification of the extracted nucleic acid.

Although a few preferred embodiments of the present invention have been shown and described, it will be appreciated by those skilled in the art that various changes and modifications might be made without departing from the scope of the invention, as defined in the appended claims.

**Brief Description of Drawings**

For a better understanding of the method and apparatus described herein, and to show how embodiments of the same may be carried into effect, reference will now be made, by way of example only, to the accompanying diagrammatic drawings in which:

Figure 1 is a flowchart of a method for determining whether an individual read in a sample is pathogenic;
Figure 2 is an extract from a taxonomic path;
Figures 3a to 3e are flowcharts of various methods for determining whether a sample contains a pathogen using the method of Figure 1;
Figures 4a and 4b are schematic illustrations of a pathogen genome showing the locations of read which are classified as pathogenic within the genome;
Figure 5 is a schematic block diagram for implementing the methods;
Figure 6a plot count against ANI score for ANI score between the bacillus anthracis reference sequence compared to every other complete reference sequence in the bacillus genus;
Figure 6b plots a distribution on the Figure 6a plot;
Figure 7a plots the number of bacillus anthracis reads which were classified as bacillus anthracis; and
Figure 7b plots the number of bacillus thuringiensis reads which were classified as bacillus thuringiensis.

**Detailed Description of Drawings**

Generally, we describe a method and system for detecting pathogens in a sample, particularly an air sample. The method is designed to run in real-time alongside sequencing processing of the sample so that results are quickly available to a user. A confidence score is used as part of the decision-making process to decide if an individual read represents a genuine match to a pathogen. As will be appreciated, the method needs to be very sensitive to pathogens and the decision-making process needs to have an extremely low false positive rate.

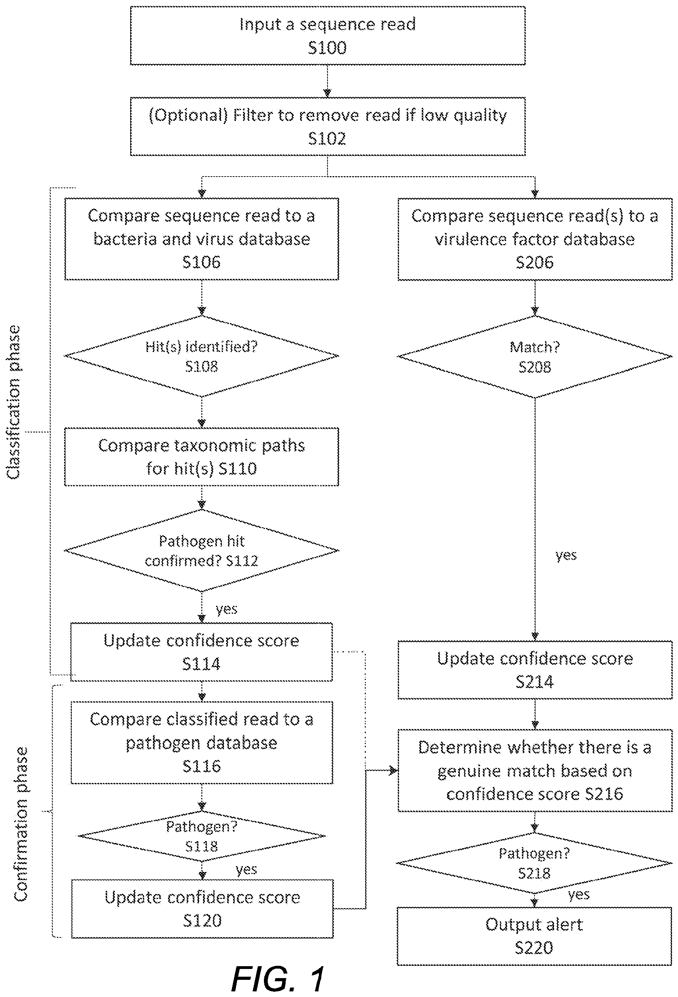

Figure 1 is a flowchart illustrating the steps in the method for detecting pathogens which may be termed an Airscreen pipeline, particularly when processing environmental air samples. In this flowchart, the steps are carried out for each sequence read which is obtained from the sample. In a first step, a sequence read is input (step S100). Sequence reads may be converted from a signal to a sequence (i.e. base called) by any suitable method, e.g. MinKNOW or Guppy software produced by Oxford Nanopore. The sequence reads may be obtained by any suitable method. In general, a sequence read will be obtained by sequencing a sample which may comprise a pathogen. The sample may be obtained from the environment, for example air, water or soil or may be a medical sample, e.g. In order to obtain a sample from the air, a specialised air-sampler device may be used such as the Coriolis Micro air sampler. These devices may use a filter to capture pathogens circulating in the air, the pathogens may be removed from the filter and further analysed to determine the sequence of their nucleic acid. Alternatively, the device may not use a filter, for example the Coriolis Micro air sampler captures a sample into a liquid. Methods are known in the art for isolating and sequencing nucleic acid from a sample which may comprise pathogens. These methods may comprise extracting the nucleic acid from the sample and amplifying the nucleic acid followed by sequencing. Extraction of nucleic acid may be performed by organic extraction or solid phase extraction. Amplification of nucleic acid may be performed by PCR amplification or whole genome amplification. Nucleic acid sequencing may be performed by any suitable whole genome shotgun sequencing method.

In a second optional step, the quality of the input sequence read may be determined and if the quality is not sufficient, the input sequence read may be removed (step S102). This step may be considered to be a filtering or pre-filtering step and may be carried out by a filter module which may apply criteria based on length and/or quality of the sequence read. Reads which are shorter than a length threshold (typically 500 base pairs) or have a mean quality value below a quality threshold (typically lower than 9) are removed. The mean quality value may be calculated by associating each letter of DNA with a quality score and then taking the mean. It will be appreciated that the length and quality thresholds are configurable by a user. If the input sequence read is not to be processed, the method loops back to the first step to input another sequence read.

As shown, each input sequence read which is to be processed is generally subject to two separate analysis tracks which may be implemented in parallel (as shown) or sequentially. The first analysis track comprises two phases: a classification phase and a confirmation phase. The second analysis track is optional and as explained below may be used to verify or supplement the analysis performed in the first analysis track.

In the classification phase, the first step is to compare the sequenced read to a first database or library (step S106) of sequences, e.g. the NCBI's nt database. The first database may comprise sequences from all domains (e.g. plant, animal, bacteria and viruses), sequences from selected domains (e.g. bacterial and virus sequences) or may be limited to just sequences for pathogens, e.g. bacterial, fungal and/or viral pathogen sequences. The comparison may be done using any suitable algorithm or tool which finds matches between the input sequence read (also termed a query or query sequence) and the sequences stored in the database. For example, the basic local alignment search tool (BLAST) may be used or a k-mer searching algorithm such as Kraken may be used. BLAST is described for example in "Basic Local Alignment Search Tool" by Altschul et al published in Journal of Molecule Biology 1009, Oct 5; 215(3):403-10. BLAST implements a seed and extend algorithm to find matches between the query sequence and target sequences in the database. In a first seed step, the tool attempts to identify short matches between the query and target sequences, in other words to find matching sub-sequences in the two sequences. In the second extend step, having found a short match, the tool attempts to enlarge the match by looking either side of the matching sub-sequence. Kraken is described for example in "Improved metagenomic analysis with Kraken 2" by Wood et al in Genome Biol, 2019; 20(1): 257.

As shown, the next step is to determine whether any hit(s) have been identified (step S108). A hit may be considered to be found when the query sequence resembles a sequence in the first database. When the database comprises sequences from both pathogens and non-pathogens, the query sequence may resemble a sequence for a pathogen or a sequence for a non-pathogen. The matching threshold (i.e. the minimum threshold which needs to be met for a query sequence to be considered to resemble a target sequence) may be configured by the user. For each input sequence read there is a resulting set of between 0 and many hits. The number of hits within each set may be refined by identifying a highest scoring hit within the set and selecting all hits having a score within a score threshold (e.g. 90%) of the highest score. The number within the set may be limited to a maximum number (e.g. 20 hits). The score threshold and the maximum number may be configurable by the user.

For example, BLAST or k-mer matching algorithms may be used in step S108. BLAST implements a number of algorithms and for the purposes described in this application, suitable algorithms include MegaBLAST and BLASTN algorithms which compare nucleotide sequences (the 4 letters A, C, G, T of DNA) with nucleotide databases. By contrast, BLASTP and BLAST X which are used in some prior art papers compare to protein database which are composed of amino acids (represented by 20 letters). Such algorithms are typically slower and may thus not be suitable for this first comparison. More information on some of the BLAST algorithms are found for example in "BLAST: at the core of a powerful and diverse set of sequence analysis tools" by McGinnis et al published in Nucleic Acids Research, Volume 32, Issue suppl_2, 1 July 2004, Pages W20-W25. As another comparison, it is noted that MetaPhlAn2 works by identifying specific marker genes which is a speedier process and may be useful to get an idea of the approximate makeup of the whole community. However, MetaPhlAn2 is typically not as accurate or as specific an approach as one based on the whole genome and is thus likely to be unreliable for pathogen identification

For example, using suitable BLAST algorithms, a sufficiently close match between sub-sequences is called a high-scoring pair (HSP), and a query sequence is said to "hit" a target sequence if they share one or more HSPs. Each HSP may be associated with a score which may be termed a "bitscore" and which is based on the similarity of the sub-sequences. The bitscore may be used to identify the highest scoring hit as described above. When comparing the query sequence with target sequences in a larger database, there is a higher likelihood that some good matches with occur due to chance. Thus, each HSP is also associated with an "E-value" representing the expected number of matches with a similar or higher score that are likely to be found in a database of the same size. For example, an E-value of 0.05 means that a match is expected due to chance in 1 in 20 similar searches, whereas an E-value of 2.0 means that two matches would be expected due to chance for a similar search. Merely as an example, the user may configure the E-value threshold or may choose to accept the typical minimum E-value threshold of 0.001. Once the E-value threshold is configured, only hits with E-values smaller than the E-Value threshold will be reported. In other words, by configuring the E-value threshold, the user may be considered to be configuring the matching threshold described above.

Once the set of hits have been identified, the next step is to compare taxonomic paths of each hit within the step (step S110) to identify a taxon for the input sequence read. In other words, each input sequence read is assigned to a single taxon. Each taxonomic path may be determined by reference to a standard taxonomy, e.g. the NCBI taxonomy. Such taxonomies may result in paths like: root, cellular organisms, bacteria, proteobacteria, Gammaproteobacteria, Enterobacterales, Enterobacteriaceae, Klebsiella, Klebsiella pneumoniae. The assignment to a taxa may be done using any suitable algorithm or tool, for example a lowest common ancestor (LCA) algorithm. Using BLAST followed by LCA may be termed BLAST/LCA.

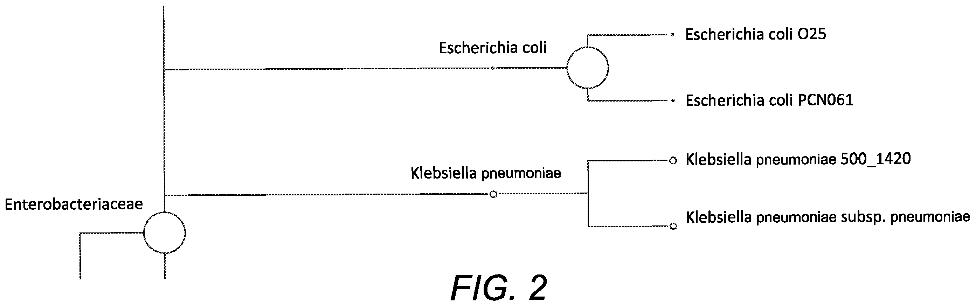

Figure 2 illustrates an extract from a taxonomic path which may be used in this step. The taxonomic paths for all identified hits are compared to determine a common ancestor. For example, this may be done by starting at the root node and working down the taxonomic path and comparing each node, "root", "cellular organisms", "bacteria" and so on until the paths diverge. The last node which each of the hits have in common before the paths diverge is the lowest common ancestor for the set of hits and the read is assigned to this taxon. With reference to Figure 2, if a read returned hits to Escherichia coli O25 and Escherichia coli PCN061 (two strains of E. coli), the Lowest Common Ancestor would be Escherichia coli. However, if a read returned hits to "Escherichia coli" (species) and "Klebsiella pneumoniae" (species), then the Lowest Common Ancestor would be Enterobacteriaceae (family).

Once a read has been assigned to a taxon, the next step S112 is to determine whether a pathogen has been identified. This may be done by comparing the assigned taxon to a list of pathogen taxa to determine whether there is a match. A read which is assigned to a taxon which matches (i.e. corresponds to) a pathogen taxon is classified as a potential pathogen. A read which is not assigned to a taxon which matches (i.e. corresponds to) a pathogen taxon may be classified as a non-pathogen or may be unclassified. In addition to matching the assigned taxon to a pathogen taxon in the list, various criteria may be required to be met before the input sequence read is classified as a putative or potential pathogen hit. The criteria may comprise one or more of an identity value exceeding an identity threshold, a query cover value exceeding a query cover threshold, a combined score based on the identity value and the query cover value exceeding a combined score threshold and a minimum length of a matching sequence. The identity value may be the amount (e.g. percentage) of the sequence read which matches a target sequence and an example identity threshold is 70%. The query cover value may be the amount (e.g. percentage) of the overall length of the sequence read which matches a target sequence and an example query cover threshold is 40%. When the identity value and query cover value are summed together to give a combined score, the combined score threshold may be 120%, as an example. Merely as an example, the minimum length may be defined as 100 base pairs. When a highest scoring hit is identified in each set of hits, e.g. as described above with reference to BLAST, the identity value, the query cover value and the minimum length may be determined for the highest scoring hit. It will be appreciated that each of these thresholds is configurable.

As explained in more detail below, a confidence score may be used to determine if each individual read is a genuine match to a pathogen. Accordingly, when a putative or potential pathogen hit is identified during the classification phase, the confidence score may be updated (step S114), for example by increasing the confidence score by a fixed amount. If a putative pathogen is not identified, no change to the confidence score is made. This step may be done before the confirmation phase or at the same time as the confirmation phase.

The confirmation phase is a phase of further analysis of any input sequence read which is classified as a potential pathogen in the classification phase. The confirmation phase may also be termed a second classification phase which is completed after the first classification stage has identified a potential pathogen. Any input sequence reads which are not classified as a potential pathogen are not processed in the confirmation phase and another input sequence read is then processed. The result of the classification of the input sequence read as a non-pathogen may be output. When the confirmation phase is initiated, the first step S116 is to compare the read to a second database. The comparison may be done using any suitable algorithm or tool which finds matches between the input sequence read (also termed a query or query sequence) and the target sequences stored in the database. The confirmation phase uses a second classification algorithm which is significantly more accurate than the first classification algorithm used in the classification phrase.

An example of a suitable algorithm is MetaMaps which is described for example in "MetaMaps - Strain-level metagenomic assignment and compositional estimation for long reads" by Dilthey et al published in Nature Communications doi: 10.1038/s41467-019-10934-2. MetaMaps is a sequence classification algorithm which is designed for long reads. MetaMaps implements a two-stage analysis procedure. In a first stage, a list of possible mapping locations for each long read is generated using a minimizer based approximate mapping strategy. In a second stage, each mapping location is scored probabilistically using a model described in the paper and a total sample composition is estimated.

The next step S118 is to classify the potential pathogen identified in the first classification phase as a pathogen or not as a pathogen. When a putative pathogen hit is confirmed as a pathogen during the confirmation phase, the confidence score may be updated (step S120), for example by increasing the confidence score by a fixed amount. If the putative pathogen is not confirmed as a pathogen, no change to the confidence score is made.

Using a second classification algorithm which is much more accurate than the first classification algorithm means that classification by the second algorithm would be significantly slower, e.g. by orders of magnitude, if the classification was done using the same parameters, e.g. same database. This may be mitigated by reducing the number of comparisons which are made by the second classification algorithm. As described above, only sequence reads which may be pathogens are processed by the second classification algorithm. A further time saving may be made by reducing the search space of target sequences. The second database to which the read is compared may be selected based on the potential pathogen which has been identified. For example, if the classification phase indicates that "bacillus anthracis" has been potentially detected, the bacillus database may be used. It will be appreciated that only some of the bacilli in the bacillus database are pathogens and the second database may be considered to be a database which discriminates between pathogens and harmless relatives. In other words, the read is compared to a genus-level database. Such genus-level databases may be created from known databases such as RefSeq as described below.

The tables below compare the performance of BLAST/LCA and MetaMaps which are examples of the first and second classification algorithms which may be used in the classification and confirmation phases, respectively. Four pathogens from a simulated read set are selected for the comparison: Bacillus anthracis, Clostridium botulinum, Clostridium perfringens and Yersinia pestis. For the true positive rate, each read classified as a potential pathogen was compared to the respective genus-level database when using MetaMaps, e.g. the Bacillus database, the Clostridium database and the Yersinia database, respectively. A strain related to each of these four pathogens is selected to determine the false positive rate: Bacillus thuringiensis, Clostridium drakei, Clostridium saccharoperbutylacetonicum and Yersinia pseudotuberculosis.

**True Positives (TP)**

| Pathogen | Bacillus anthracis | Clostridium botulinum | Clostridium perfringens | Yersinia pestis |
|---|---|---|---|---|
| No. of reads classified as pathogen by Blast/LCA | 8355 | 8976 | 8977 | 7852 |
| No. of Blast/LCA pathogen reads classified as pathogen by MetaMaps | 8334 | 8942 | 8950 | 7852 |
| % of Blast/LCA pathogen reads confirmed by Meta-Maps | 99.75 | 99.62 | 99.7 | 100 |
| % reads classified as pathogen by both tools | 83.34 | 89.42 | 89.5 | 78.52 |

It is noted that the true positive rates shown above in which the appropriate genus-level database was employed to classify reads from Bacillus anthracis, Clostridium botulinum, Clostridium perfringens are identical to those for the MetaMaps default database. A small improvement in true positive rates is seen for Yersinia pestis, with the MetaMaps Yersinia genus-level database now classifying all Blast/LCA pathogen reads as pathogen. For the false positive rates shown below it is noted that a higher proportion of Yersinia pseudo tuberculosis reads incorrectly classified as Yersinia pestis reads by the first classification algorithm (BLAST/LCA) were correctly classified as relative reads via MetaMaps' Yersinia database

**False positives (FP)**

| Relative | Bacillus thuringiensis | Clostridium drakei | Clostridium saccharoperbutylacetonicum | Yersinia pseudo tuberculosis |
|---|---|---|---|---|
| No. of reads classified as pathogen by Blast/LCA | 68 | 0 | 0 | 348 |
| No. classified as pathogen by MetaMaps | 0 | 0 | 0 | 0 |
| No. classified correctly to relative by MetaMaps | 67 | 0 | 0 | 344 |
| No. classified to genus or higher by MetaMaps | 1 | 0 | 0 | 4 |
| % FP confirmed by Meta-Maps | 0% | 0% | 0% | 0% |
| %FP rejected by Meta-Maps | 100% | 100% | 100% | 100% |

Returning to Figure 1, there is an optional analysis phase which is shown as occurring at the same time to the classification phase, although it will be appreciated that it may also be run after or before one or both of the classification and confirmation phases. In step S206, a sequence read which has not been removed by the optional filtering step is compared to target sequences in a virulence factor database such as the VFDB described in more detail below. If a match between the input sequence read and one or more target sequences is found at step S208, the confidence score is updated at step S214. If no match is found, no change to the confidence score is made.

After each of the classification phase, the confirmation phase and the optional analysis phase have been completed, the results of each phase are input to a decision-making module to determine whether there is a genuine match between the input sequence read and a pathogen (step S216). The decision-making module may use a confidence scoring mechanism to decide whether an individual read represents a genuine match to a pathogen, i.e. is to be classified as pathogenic or non-pathogenic. As described above, the confidence score is updated (i.e. incremented) when a pathogen is identified by the first classification algorithm (step S114), when the identified pathogen is confirmed by the second classification algorithm (step S120) and when there is a match for a virulence factor (step S214). The increment to the confidence score may be the same for each result or may be weighted so that some results are more important in determining whether there is a genuine match to a pathogen. For example, the confidence score may be incremented by the same amount (e.g. two) when a pathogen is identified by the first classification algorithm (step S114) and when the identified pathogen is confirmed by the second classification algorithm (step S120). The confidence score may be incremented by a lower amount (e.g. one) when there is a match for a virulence factor (step S214). In other words, the hits using the first and second classification algorithms are weighted more heavily than the hit on the virulence factor database.

A read is classified as pathogenic when the confidence score which is received by the decision-making module is greater than or equal to a configurable confidence score threshold and classified as non-pathogenic when the confidence score is lower than the confidence score threshold. The confidence score threshold may be configured so that a genuine match is only output when at least one of the two classification algorithms results in a hit to a pathogen and there is a hit on the virulence factor database. For example, using the simple scoring above in which the confidence score is incremented by two when a pathogen is identified by the first or second classification algorithms and by one when a virulence factor is confirmed, the confidence score threshold may be set at three.

Optionally other criteria may also need to be met for a read to be marked as pathogenic. The other criteria may include criteria for the result of the first classification algorithm, including for example the identity value exceeding an identity threshold, the query cover value exceeding a query cover threshold, and/or a minimum length for the matching sequence.

When the decision-making module classifies a read as pathogenic at step S218, an alert may be output to a user as shown at step S220. The alert may be an audio or visual message which is issued to a user, for example by displaying the message on a display screen. In addition to issuing an alert, a summary report which includes the classifications from both the first and second classification algorithms may be sent to a third party (e.g. a third-party network) for further processing such as comparison with results obtained from other types of sensors, e.g. chemical, biological and radiological sensors.

Figure 1 shows the process for classifying each individual read in the plurality of reads collected from a sample as pathogenic or non-pathogenic. Although Figure 1 shows an alert being generated when a single read is classified as a pathogen, typically, it will not be sufficient for just a single read to be classified as a pathogen for sensitivity requirements to be met to trigger an alert. Accordingly, a simple way to improve sensitivity may be to repeat the process of Figure 1 for multiple reads which have been extracted from the sample. An alert may only be triggered when more than a threshold number of reads (e.g. three) are classified as a particular pathogen.

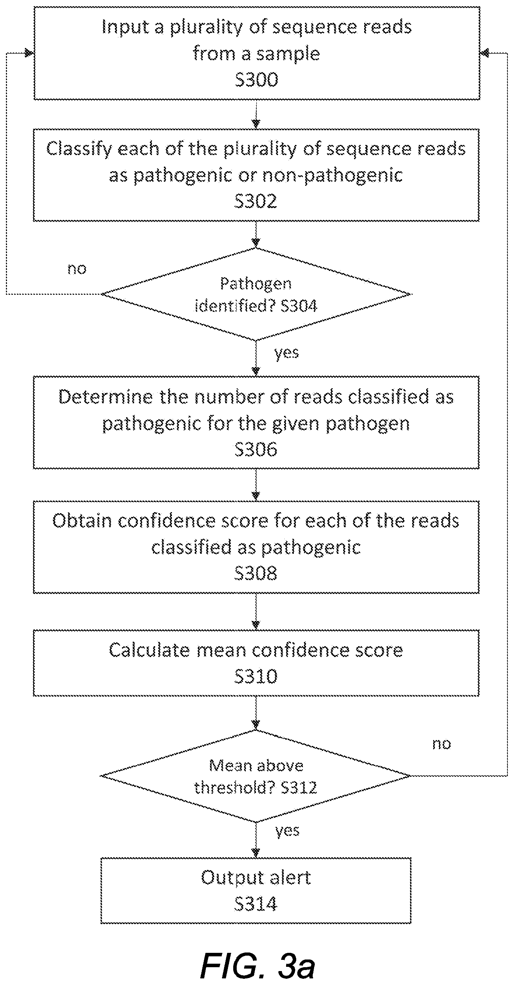

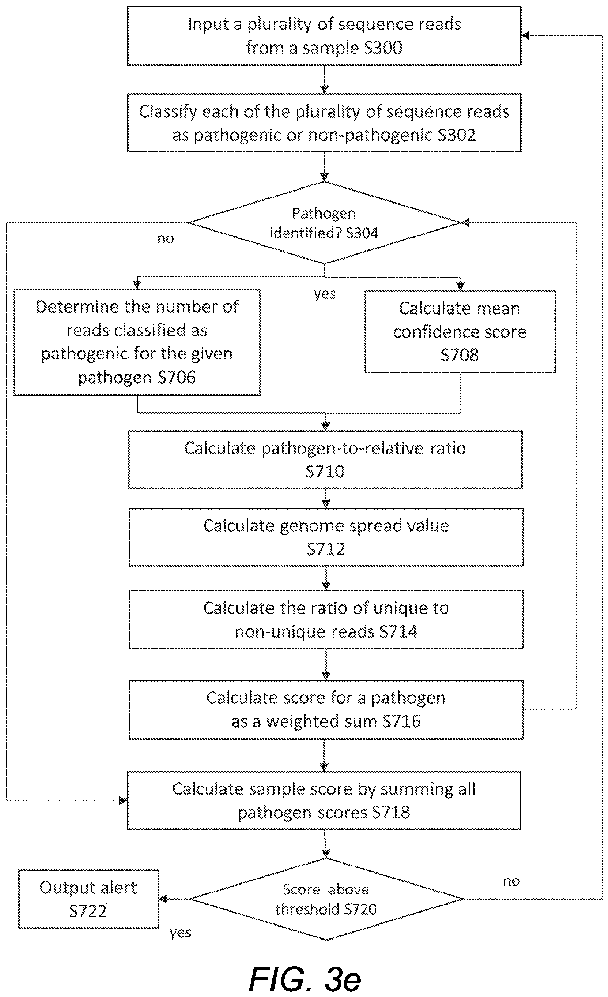

Figures 3a to 3d are flowcharts showing how the results from each individual read can be combined in different ways to improve the overall classification for each sample. It will be appreciated that one or more of the processes in Figures 3a to 3d may be used together as shown in Figure 3e.

Figure 3a illustrates a process which can be used to determine a mean confidence score when there is more than one read classified as a particular pathogen. A plurality of sequence reads from a sample are input in a first step S300. Each of the plurality of sequence reads is classified as pathogenic or non-pathogenic (step S302) using the method described in Figure 1. If no pathogens are identified in the sample at step S304, the process can begin again with a new sample.

When at least one pathogen is identified, the following sequence of steps is carried out for each pathogen which has been identified. As shown at step S306, we determine (or obtain) the number of reads $n_p$ which are classified as pathogenic for the pathogen being considered. For each of these reads which have been classified as pathogenic, the confidence score for each read is obtained (step S308). The confidence score is the score determined by the process of Figure 1. The mean (or average) confidence score $s_{mean}$ is then calculated (step S310). The mean confidence score is then compared to a mean confidence score threshold (step S312). When the mean confidence score is greater than or equal to the mean confidence score threshold, an alert is output (step S314). When the mean confidence score is lower than the mean confidence score threshold, the process can begin again with a new sample.

The mean confidence score threshold can be configurable to improve accuracy. Merely as an example, the mean confidence score threshold may be higher than the confidence score threshold used to determine whether an individual read is to be classified as pathogenic. For example, in the scoring example used above, the mean confidence score threshold may be set to four when the confidence score threshold is set to three.

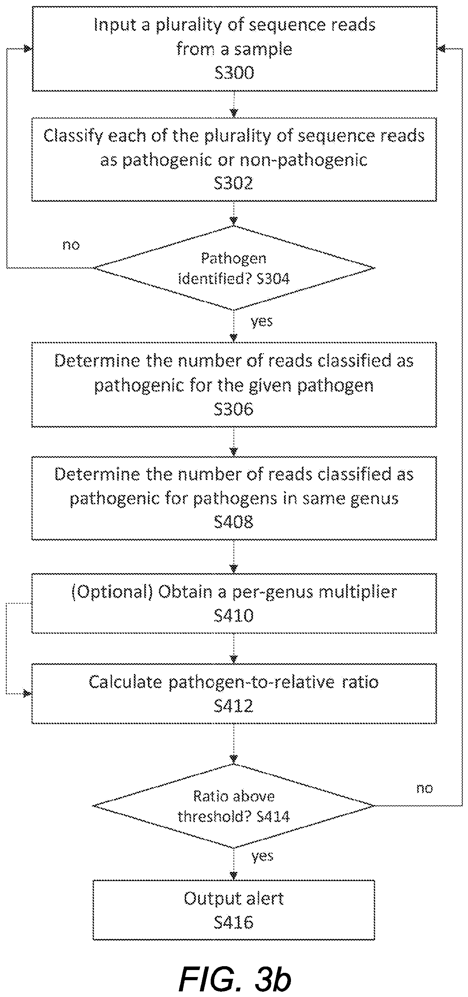

Figure 3b illustrates a process in which the number of pathogenic reads for a particular pathogen is compared to the number of reads classified to the same genus as the particular pathogen to determine a ratio which may be termed a pathogen to relative ratio. The reason for such a comparison is that the presence of bacteria from the same genus as the pathogen decreases confidence in the correct classification of the pathogen. For example, if many sequence reads are classified as *bacillus thuringiensis,* it is more likely that classifications of a small number of sequence reads as *bacillus anthracis* are misclassifications than true pathogen detection.

The first few steps of Figure 3b overlap with those of Figure 3a. In other words, a plurality of sequence reads from a sample are input in a first step S300. Each of the plurality of sequence reads is classified as pathogenic or non-pathogenic (step S302) using the method described in Figure 1. When at least one pathogen is identified at step S304, the following sequence of steps is carried out for each pathogen which has been identified. As shown at step S306, we determine (or obtain) the number of reads $n_p$ which are classified as pathogenic for the particular pathogen. The next step diverges from Figure 3a and as shown at step S408, we determine (or obtain) the number of reads $n_g$ which are classified in the same genus as the particular pathogen.

For some pathogens, there is a greater likelihood of naturally occurring relatives in the same genus than there is for other pathogens. This means that when there is a large background of the same genus, the pathogen-to-relative ratio will be very low. A pathogen would not then be detected unless it was present at unfeasibly high levels. Accordingly, at step S410, an optional per-genus multiplier $m_g$ may be provided to mitigate against this problem.

The pathogen-to-relative ratio $R_p$ is then calculated at step S412. The pathogen-to-relative ratio $R_p$ may be calculated simply as:

$$R_p = \frac{n_p}{n_g}$$

where $n_p$ is the number of pathogen reads and $n_g$ is the number of reads classified to the same genus as the pathogen.

Alternatively, when the per-genus multiplier is obtained, the pathogen-to-relative ratio $R_p$ may be calculated as

$$R_p = \frac{n_p}{m_g(n_g - n_p) + n_p}$$

where $n_p$ is the number of pathogen reads, $n_g$ is the number of reads classified to the same genus as the pathogen and $m_g$ is the per-genus multiplier. The per-genus multiplier may be set to 1 as a default value. Increasing the value of the per-genus multiplier, increases the importance of the background and thus decreases the value of the pathogen-to-relative ratio. Decreasing the value of the per-genus multiplier, decreases the importance of the background and thus increases the value of the pathogen-to-relative ratio. Accordingly, a value below 1 may be used when there is a large background of the same genus.

A pathogen-to-relative ratio $R_p$ having a value close to zero indicates that the pathogen reads are a small part of a large assignment to the genus. A pathogen-to-relative ratio $R_p$ value closer to one indicates that the pathogen reads make up a significant proportion of reads assigned to the genus. The pathogen-to-relative ratio $R_p$ is compared to a pathogen-to-relative threshold at step S414. Merely as an example, the pathogen-to-relative threshold may be set at 0.5 but this is configurable to provide the desired accuracy of the method. When the pathogen-to-relative ratio $R_p$ is above or equal to the pathogen-to-relative threshold, an alert is output step S416.

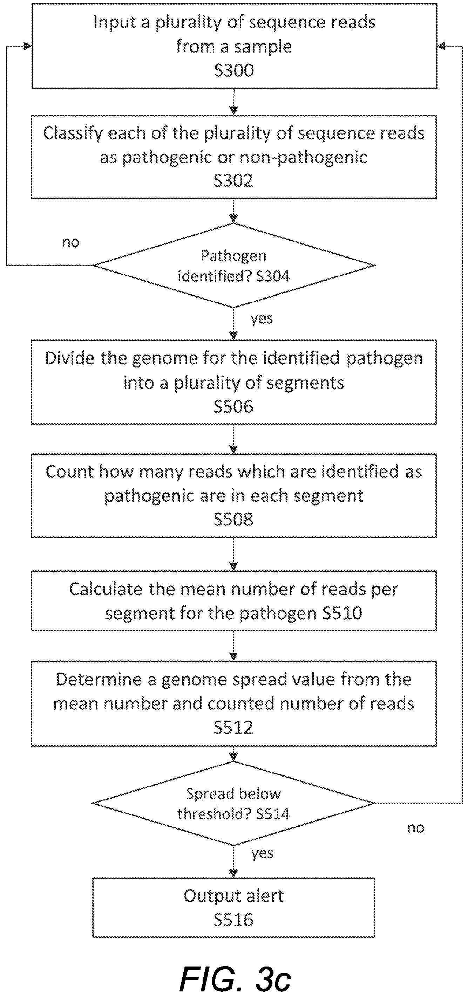

Figure 3c illustrates a process in which the spread across the genome of reads which are classified as pathogenic is considered. When the reads which are classified as pathogenic are concentrated in a specific region of the genome of the pathogen, it is more likely that these reads could also belong to harmless relatives of the same genus because they represent sequence in common and hence the sample may be incorrectly classified as containing a pathogen. When the reads which are classified as pathogenic are spread over the genome of the pathogen, a classification of the sample as containing a pathogen is more likely to be correct.

The first few steps of Figure 3c overlap with those of Figure 3a. In other words, a plurality of sequence reads from a sample are input in a first step S300. Each of the plurality of sequence reads is classified as pathogenic or non-pathogenic (step S302) using the method described in Figure 1. When at least one pathogen is identified at step S304, the following sequence of steps is carried out for each pathogen which has been identified. The genome for the identified pathogen is first divided into a plurality of segments (step S506). The segments may be termed bins and may be relatively large. Merely as an example, the genome may be divided into say 10 bins which for a typical bacterial genome having 2 million to 6 million base pairs of sequence means that each bin represents a few hundred thousand base pairs of sequence.

The next step is then to determine how many bins contain reads which have been classified as pathogenic and count how many reads are in each bin (step S508). When a read overlaps with two bins, the read may be assigned to the bin with which it has the greatest overlap or it may be assigned to both bins. Given a total of $n$ bins and $m$ reads which are hits, the mean number $N_m$ of reads per segment (bin) may calculated at step S510 from:

$$N_m = \frac{m}{n}$$

The mean number $N_m$ of reads per segment may be considered to be the theoretical number of hits per bin and is combined with the actual number of reads in each bin to determine the spread of reads across the genome. A value termed the genome spread $g_s$ may be used to represent the spread of reads across the genome. The genome spread $g_s$ may be a probability distribution with a number close to one representing a uniform distribution and a number close to zero indicating that the distribution is nothing like a uniform distribution.

One method of calculating the genome spread $g_s$ is to consider the read counts determined in step S508 to represent a probability distribution which can be compared to a predicted (or uniform) distribution for the reads across the genome. The count distribution may be compared to the uniform distribution using a distance measure, e.g. total variation distance $V_d$. A value of $V_d$ equal to 1 indicates that the count distribution is nothing like a uniform distribution and a value of $V_d$ equal to 0 indicates that the count distribution is a uniform distribution The genome spread $g_s$ may then be calculated as

$$g_s = 1 - V_d$$

The genome spread may then be compared to a genome spread threshold at step S514. When the genome spread is above or equal to the genome spread threshold, there is a good spread of reads which are classified as pathogenic across the genome and thus an alert may be outputted at step S516. When the genome spread is below the genome spread threshold, the reads are likely to be clustered in segments and thus there is more likely to be a misclassification of each read as containing a pathogen. Accordingly, no alert is output and the method may loop back to the next sample.

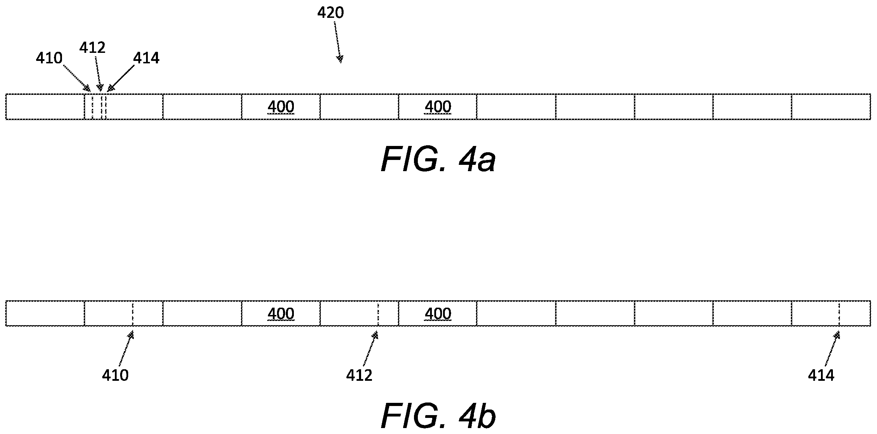

As illustrations, Figures 4a and 4b illustrate two scenarios in which three reads 410, 412, 414 within the same genome 420 are classified as pathogenic. In line with the method of Figure 3c, the genome 420 is divided into a plurality of segments 400 (in this example there are 11 segments but it will be appreciated that this is merely indicative). As shown in Figure 4a, the three reads are all within the same segment 400. By contrast, in Figure 4b, each of the three reads is in a separate segment and are well spread across the entire genome. The result of Figure 4b is thus more likely to be a correct identification of the pathogen within the overall sample. Using the method of Figure 3c, the sample in Figure 4a will not be classified as containing a pathogen.

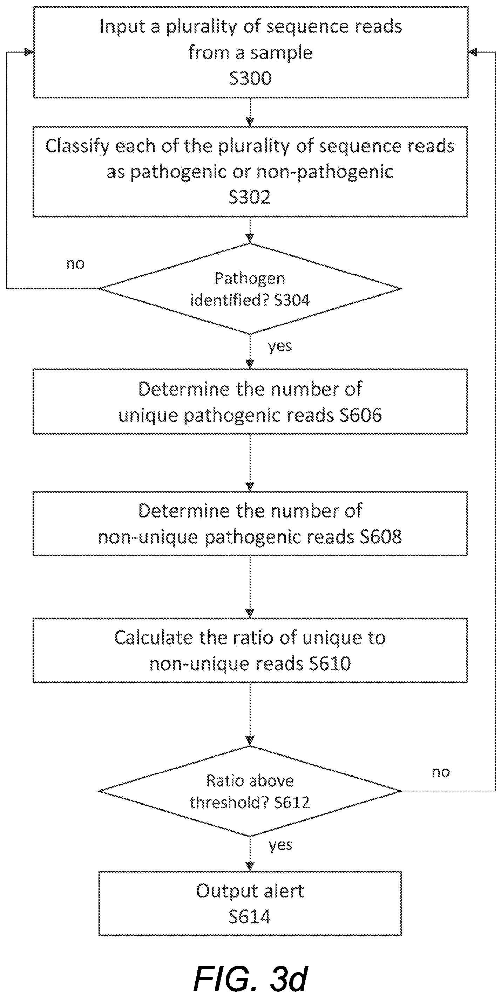

Figure 3d illustrates a process in which the uniqueness of each read which is classified as pathogenic is considered. A unique hit is one for which the read only matches a target sequence within the pathogen species and is not a good match to similar sequences within other species. A hit to a part of the genome which is a unique sequence may be considered to be a more reliable classification as a real pathogen than a hit to a part of the genome which has a similarity to other species in the same genus.

The first few steps of Figure 3d overlap with those of Figure 3a. In other words, a plurality of sequence reads from a sample are input in a first step S300. Each of the plurality of sequence reads is classified as pathogenic or non-pathogenic (step S302) using the method described in Figure 1. When at least one pathogen is identified at step S304, the following sequence of steps is carried out for each pathogen which has been identified.

In step S606, the number of reads which have been classified as pathogenic and which are unique is determined. In step S608, the number of reads which have been classified as pathogenic and which are not unique is determined. The Lowest Common Ancestor algorithm which is used in the process described in Figure 1 already takes "good hits" into account when assigning reads to taxa. Accordingly, when the same measure of "goodness" is used for steps S606 and S608, all reads will be considered as "unique" hits. Thus, when determining the number of unique and non-unique pathogenic reads in step S606 and S608, the criteria used in Figure 1 to determine the number of hits within each set of hits need to be relaxed. For example, the score threshold may be reduced (e.g. from 90% to 80%) or the number of hits to be considered may be increased (e.g. from 20 to 30).

In step S610, the uniqueness ratio is calculated as:

$$U_p = \frac{n_u}{n_n}$$

where $n_u$ is the number of unique pathogenic reads and $n_n$ is the number of non-unique pathogenic reads. The uniqueness ratio may be compared to a uniqueness threshold at step S612 and an alert may be output step S614 when the uniqueness ratio is greater than or equal to the uniqueness threshold. The uniqueness threshold is configurable to provide the desired accuracy of the method.

As an alternative, or in addition to using the uniqueness ratio, when a read is identified as being unique, the confidence score for that individual read may be incremented by a fixed amount. For example, in the example above in which outcomes are scored as two or one respectively, the confidence score may be incremented by three when a pathogenic read is unique. The read may be identified as being unique by determining which target sequences within a genome of a pathogen are unique. This could be done when building the databases which are used in the classification phases. As an example, the uniqueness of each target sequence may be determined using a tool called genomic origin through taxonomic challenge (GOTTCHA) described for example in "Accurate read-based metagenome characterisation using a hierarchical suite of unique signatures" by Freitas et al published in Nucleic Acids Research, Volume 43, Issue 10, 26 May 2015. The GOTTCHA tool introduces the idea of identifying unique parts of a genome at different taxonomic levels. For example, it can compare all genomes of a specific genus and identify the unique parts of each genome.

The methods described in Figures 3a to 3d may be used individually to classify a sample or may be combined as shown in Figure 3e to further improve accuracy of the classification. The first few steps of Figure 3e overlap with those of Figure 3a. In other words, a plurality of sequence reads from a sample are input in a first step S300. Each of the plurality of sequence reads is classified as pathogenic or non-pathogenic (step S302) using the method described in Figure 1. When at least one pathogen is identified at step S304, the following sequence of steps is carried out for each pathogen which has been identified.

The number of reads $n_p$ classified as pathogenic for each particular pathogen is determined at step S706 and the mean confidence score per read $s_{mean}$ is calculated at step S708. Both these steps may be carried out as described in Figure 3a. The pathogen to relative ratio $R_p$ is then calculated at step S710 as described in Figure 3b. At step S712, the genome spread value $s_g$ is calculated as described in Figure 3c. At step S714, the ratio of unique to non-unique reads $U_p$ is calculated as described in Figure 3d. It will be appreciated that although steps S706 to S714 are shown sequentially in Figure 3e, they may be carried out in any order and may be carried out simultaneously or sequentially.

An overall score for the particular pathogen $S_P$ may then be calculated in step S716 as a weighted sum of the ratios and scores determined previously for the particular pathogen. For example, the score $S_P$ may be calculated as:

$$S_P = n_p \, s_{mean} \, (1 + \alpha R_p + \beta s_g + \delta U_p)$$

where $n_p$ is the number of pathogen reads, $s_{mean}$ is the mean pathogen per-read score, $R_p$ Is the pathogen-to-relative ratio, $s_g$ is the genome spread value, $U_p$ is the ratio of unique hits to total hits and $\alpha$, $\beta$, $\delta$ are constants used to weight the impact of the pathogen-to-relative ratio, the genome spread value and the ratio of unique hits to total hits, respectively. $\alpha$, $\beta$, $\delta$ may be initially set to 1 (i.e. equal weighting) but may be configured as required to improve accuracy. The terms $n_p$ and $s_{mean}$ act as multipliers for the weighted sum.

Once the score for a particular pathogen has been calculated, as indicated the process may loop back to step S304 to determine if there is another pathogen which has been identified. Steps S706 to S716 are then repeated for another particular pathogen if there is one. This loop is repeated until there are no further pathogens. The process then moves to step S718 in which an overall score for the sample is obtained by summing all the pathogen scores, i.e.

$$S_S = \sum_{i=1}^{q} S_p$$

where $S_P$ is the score for a particular pathogen, q is the number of pathogens identified in the sample and $S_S$ is the overall score for the sample.

The overall sample score may then be compared to a sample threshold at step S720 and if the sample score is above or equal to a sample score threshold, an alert may be output (step S722). The sample score threshold can be configurable to improve accuracy. If no pathogen is detected in the sample, the process may loop back to the next input sample. The process operates in real-time so that an environment may be constantly monitored for the presence of pathogens.

Merely as example of how the method of Figure 3e may be implemented, consider two scenarios in which a sample has been processed using the method of Figure 1 and three input sequence reads have been classified and then confirmed as a pathogen (i.e. classified by both the first and second classification methods - e.g. BLAST and MetaMaps - as a pathogen). In these scenarios, $n_p$ is 3, $s_{mean}$ is 4. There are no other pathogens identified. The table below compares the scores for two different scenario:

First scenario

| None of the reads hit a relative in the same genus | $R_P \cong 1$ |
| --- | --- |
| The three reads are spread across the genome | $s_g \cong 1$ |
| The threes reads are mostly unique | $U_p \cong 1$ |
| The overall score for the sample | $S_s = S_p = 3 \times 4 \times (1+1+1+1) = 48$ |

Second scenario

| Many of the reads hit a relative in the same genus | $R_P \cong 0$ |
| --- | --- |
| The three reads are clustered in one part of the genome | $s_g \cong 0$ |
| The threes reads are mostly non-unique | $U_p \cong 0$ |
| The overall score for the sample | $S_s = S_p = 3 \times 4 \times (1+0+0+0) = 12$ |

Based on the overall scores, it is unlikely that the second scenario will be classified as a sample containing a pathogen. As described above, the terms $n_p$ and $s_{mean}$ act as multipliers and thus if the number of reads which are classified in the second scenario as pathogens is significantly higher than three, the overall score will be higher and thus the sample is more likely to be classified as containing a pathogen. For example, the overall score for the sample in the second scenario may be similar to the overall score for the sample in the first scenario when four times as many reads are classified as pathogens.

The method described above was tested on real environmental samples (air sample) which have been spiked with simulated pathogen reads. Simulated read sets were created for 23 pathogens. Pathogen reads were all over 1kb in length, with a mean read size of approximately 2.5kb. For each pathogen, we spiked 500 reads into 15 real air samples collected in London during 2019. Each air sample had 52,000 reads in total. These reads were processed through the complete Airscreen pipeline, including quality filtering, BLAST searching and putative pathogen confirmation using MetaMaps genus-level databases. Though we spiked 500 reads for each pathogen, each read is analysed on its own. In other words, we are looking at individual reads rather than a community composition.

In total, 345 simulated runs of the Airscreen pipeline were completed (i.e. 23 pathogens x 15 samples). The table below provides average results per pathogen (i.e. average of 15 runs) and shows the percentage of true positives (TP), false negatives (FN), true negatives (TN) and false positives (FP):

| ID | Pathogen | TP | FN | TN | FP |
|---|---|---|---|---|---|
| BA | Bacillus anthracis | 79.7067 | 20.2933 | 99.9992 | 0.0008 |
| BM | Brucella melitensis | 74.1600 | 25.8400 | 99.9992 | 0.0008 |
| Bope | Bordetella pertussis | 90.0133 | 9.9867 | 99.9992 | 0.0008 |
| Bpse | Burkholderia pseudomallei | 88.4533 | 11.5467 | 99.9992 | 0.0008 |
| CB | Clostridium botulinum | 95.1733 | 4.8267 | 99.9992 | 0.0008 |
| Cbur | Coxiella burnetii | 94.3067 | 5.6933 | 99.9992 | 0.0008 |
| Chpn | Chlamydia pneumoniae | 97.5714 | 2.4286 | 99.9992 | 0.0008 |
| Codi | Corynebacterium diphtheriae | 87.5333 | 12.4667 | 99.9992 | 0.0008 |
| CP | Clostridium perfringens | 93.5200 | 6.4800 | 99.9992 | 0.0008 |
| Cpsi | Chlamydia psittaci | 97.5733 | 2.4267 | 99.9992 | 0.0008 |
| EC | Escherichia coli | 95.2267 | 4.7733 | 99.9992 | 0.0008 |
| FT | Francisella tularensis | 96.9867 | 3.0133 | 99.9992 | 0.0008 |
| Hain | Haemophilus influenzae | 87.0267 | 12.9733 | 99.9992 | 0.0008 |
| Lepn | Legionella pneumophila | 94.3467 | 5.6533 | 99.9992 | 0.0008 |
| Myle | Mycobacterium leprae | 85.8933 | 14.1067 | 99.9992 | 0.0008 |
| Mypn | Mycoplasma pneumoniae | 91.6800 | 8.3200 | 99.9992 | 0.0008 |
| Mytu | Mycobacterium tuberculosis | 96.1733 | 3.8267 | 99.9992 | 0.0008 |
| NeoSen | Neoricketssia sennetsu | 93.7867 | 6.2133 | 99.9992 | 0.0008 |
| Psae | Pseudomonas aeruginosa | 96.6667 | 3.3333 | 99.9992 | 0.0008 |
| RP | Rickettsia prowazekii | 97.3467 | 2.6533 | 99.9992 | 0.0008 |
| Sent | Salmonella enteric | 97.7600 | 2.2400 | 99.9992 | 0.0008 |
| VC | Vibrio cholerae | 91.1733 | 8.8267 | 99.9992 | 0.0008 |
| YP | Yersinia pestis | 78.4133 | 21.5867 | 99.9992 | 0.0008 |

When evaluating an environmental sample to assess whether a pathogen is present, it is critical not to incorrectly identify a pathogen as being present in the sample and hence trigger a false alert. In other words, the true negative rate needs to be extremely high (and the corresponding false positive very low). It is also important that if there is a pathogen present, that this should be detected with a high level of accuracy. As shown in the table above, there is a very high rate for the true negatives (99.9992%) and a correspondingly low rate for false positives (0.0008%). The true positive rate is high (e.g. above 72%) for all pathogens. The reasons for the higher rate of "False negatives" may be explained by MetaMaps being able to confirm only genus or higher level assignment rather than confirming species assignment, or in some cases, MetaMaps was unable to provide an assignment.

Looking in more detail at the low rate of "False positives," these were as a result of 6 reads out of a total of 780,000 and are detailed in the table below:

| Sample | Possible false positive species (Count) | FP alignment |
|---|---|---|
| 1 | | |
| 2 | Clostridium botulinum (1) | 1062 bp, 79% identity |
| 3 | | |

(continued)

| Sample | Possible false positive species (Count) | FP alignment |
|---|---|---|
| 4 | Clostridium botulinum (1) | 1045 bp, 80% identity |
| 5 | Haemophilus influenzae (1) | 1004 bp, 83% identity |
| 6 | | |
| 7 | Escherichia coli (1) | 1079 bp, 95% identity |
| 9 | | |
| 10 | Escherichia coli (1) | 1081 bp, 93% identity |
| 11 | | |
| 12 | Haemophilus influenzae (1) | 2165 bp, 87% identity |
| 13 | | |
| 14 | | |
| 15 | | |
| 16 | | |

Looking at the species, it is entirely possible that these are, in fact, all true positives, as the matches are good (>79-95% identity over long lengths) and each of these species can be reasonably found in environmental aerosol samples. Furthermore, as explained above, there is an option to require a threshold number of sequences to be detected before a pathogen is declared as being detected. In this example, none of the runs would have met the 3-5 read threshold that would be expected given the targeted sensitivity level. Thus, the example results show that the method provides the necessary accurate result for an environmental sample containing a small amount of pathogen.

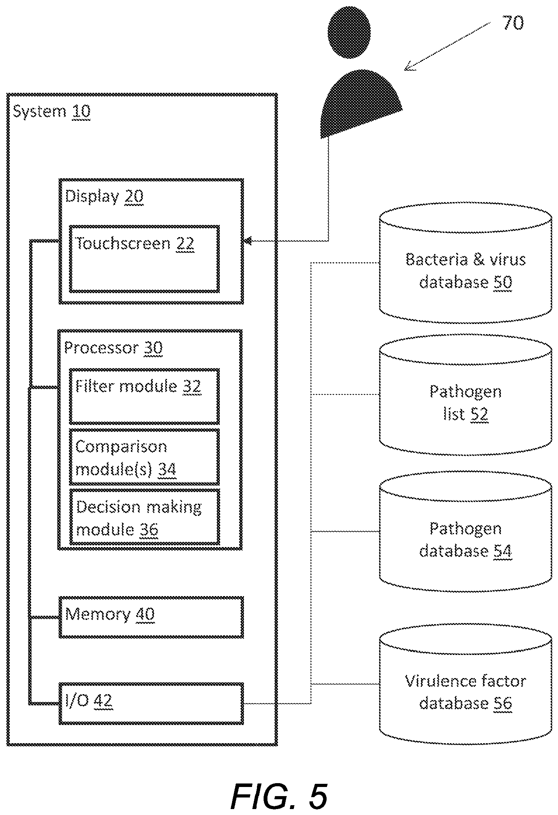

Figure 5 schematically shows a system 10 for implementing the methods described above. The system 10 is connected to a first database 50, a pathogen list 52, a second database 54 and a virulence factor database 56. It will be appreciated that the data for each type of database may be split across more than one storage device and may be stored in any suitable storage device, including the cloud. The databases may be remote (i.e. in a different location) or may be local to the system. The system 10 is also connected to a sampler 60 which extracts a sample from the air as described above. The sampler 60 may also comprise a sequencer 62 to perform nucleic acid sequencing (e.g. whole genome shotgun sequencing) on the extracted sample. It will be appreciated that the sampler 60 and sequencer 62 may also be separate devices.

The first database 50 comprises the data used for the comparison step in the classification phase (S106 of Figure 1). The first database 50 may be the nucleotide (nt) database published by the National Center for Biotechnology Information (NCBI) or any other suitable database containing nucleic acid sequences for all domains (i.e. including sequences for both pathogens and non-pathogens). Alternatively, the first database 50 may comprise only selected domains, e.g. bacteria, fungi and/or viruses (for both pathogen and non-pathogens) which can be compared with the sequence reads in the sample. For example, the NCBI's nt database may be filtered according to taxonomy identification (ID) to create a filtered database 50 which only contains nucleic acid sequences which have a taxonomy IDs of a bacteria, fungus or a virus. Such a filtered database is more tailored to the proposed process and by searching in a reduced size database, the overall search time will be reduced (perhaps by > 50%).

The pathogen list 52 is a list of pathogen taxa which is used as part of the confirmation that a pathogen has been identified. A pathogen is any substance which has the capacity to cause disease and its ability to cause disease is called pathogenicity. In this method, we are attempting to identify bacterial, fungal and/or viral pathogens and thus the pathogen list is a list of bacterial, fungal and viral pathogen taxa.

The second database 54 comprises the data used for the comparison step in the confirmation phase (S116 of Figure 1). The second database 54 is typically smaller than the first database 50 and is selected based on the pathogen taxon to which the potentially pathogenic input sequence read corresponds. For example, the second database 54 may comprise a plurality (or set) of genus-level databases for bacterial, fungal and viral pathogens and their relatives. Alternatively, the second database 54 may be a plurality of family level databases or species level databases. Each database may be built using the entire RefSeq genome set. For example, for a genus-level database and for a given genus, each genome in the RefSeq genome set is identified (e.g. using the filename), each identified sequence may then be marked and appended to a file (e.g. a FASTA file) for the genus-level. Some references may be removed for efficiency (e.g. similar to other references) or quality (e.g. known to be poor quality reference). For example, for the Bacillus database, all genomes beginning with "Bacillus_" are identified and each sequence appended to the file for the Bacillus database. A FASTA file may be used as an input to the standard database build tool for MetaMaps. One of the genus-level databases may be selected as the second database for the comparison step.

The virulence factor database (VFDB) 56 comprises the data used for the optional comparison step in the parallel track (S206 of Figure 1). The virulence factor database 56 is used to look for matches to genes known to confer virulence. Virulence provides a quantitative measure of the pathogenicity or the likelihood of causing disease. Virulence factors refer to the properties (i.e. the gene products) that enable a microorganism to establish itself on or within a host of a particular species and enhance its potential to cause disease. Virulence factors include bacterial toxins, cell surface proteins that mediate bacterial attachment, cell surface carbohydrates and proteins that protect a bacterium, and hydrolytic enzymes that may contribute to the pathogenicity of the bacterium. Any suitable virulence factor database may be used such as the one described in "VFDB2019: a comparative pathogenomic platform with an interactive web interface" by Liu et al published in Nucleic Acids Res. 47 (D1): D687-D692 and which is downloadable from www.mgc.ac.cn/VFs.

Some of the internal detail of the system 10 is shown in Figure 5. There are standard components of whichever hardware solution is deployed, including for example a display 20, a processor 30, memory 40 and an interface 42 for connecting with the databases 50, 54, 56. The display may include a touchscreen 22 or may be any other suitable display on which information may be displayed to a user 70. It will be appreciated that there may be other standard components which are omitted for clarity.

The processor 30 may process the data from the databases in any appropriate way. As illustrated, the specific processing may be completed in dedicated modules, e.g. a filter module 32, at least one comparison module 34 and a decision-making module 36. The comparison module(s) 34 may also be termed classification modules because they are implementing the comparisons against the databases to classify the sample (more specifically each individual sequence read in the sample). Merely as examples, the comparison module(s) may for example be used for steps S106, S116 and S206 in Figure 1. The decision-making module 36 may be used for the determination step S216 in Figure 1. The filter module 32 may be used for the filtering step S102 in Figure 1.

Terms such as 'component', 'module', 'processor' or 'unit' used herein may include, but are not limited to, a hardware device, such as circuitry in the form of discrete or integrated components, graphics processing units (GPUs), a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements.

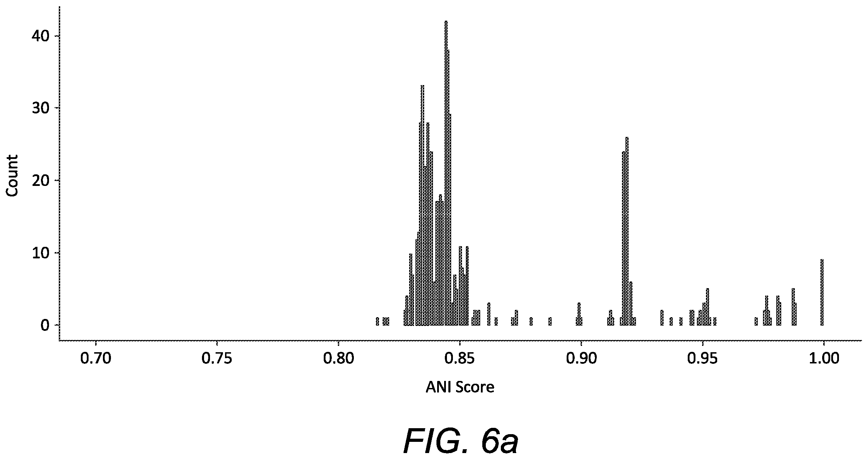

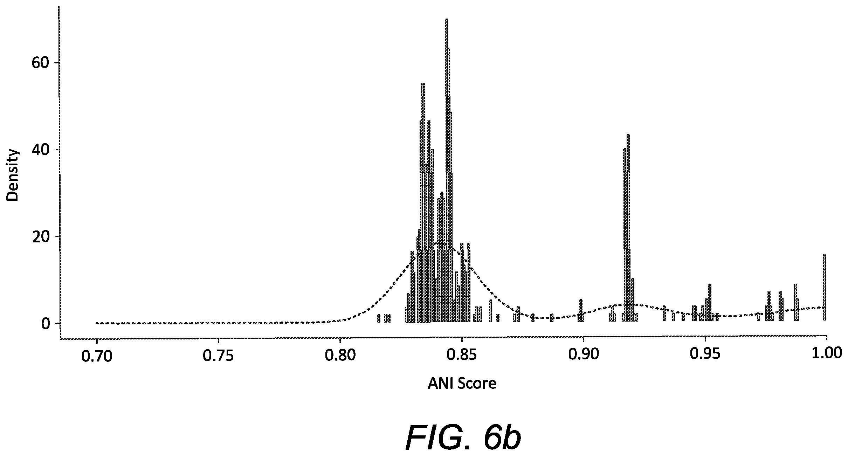

Figures 6a and 6b illustrate an alternative method for building a genus level database which may be included in the second database. In a first step, the average nucleotide identity (ANI) is calculated between a reference sequence (e.g. bacillus anthracis) and every other reference sequence in the same genus (e.g. bacillus genus). The ANI may be calculated using any suitable method. Merely as an example, the ANI score may be calculated as described in "Genomics and taxonomy in diagnostics for food security: soft-rotting enterobacterial plant pathogens" by Pritchard et al published in the Journal of the Royal Society of Chemistry in 2015. Figure 6a plots the number (i.e. count) of sequences having the same ANI score. As shown in Figure 6a, there is a cluster of sequences having ANI scores between 0.825 and 0.86. Figure 6b fits a distribution to the histogram of Figure 6b.

In a second step, all references having an ANI score within a defined (or threshold) percentage of the pathogen reference are retained. In other words, all references meeting a score criteria are retained. The defined percentage may be user-defined and may be based on the distribution of ANI scores. For example, using the distribution of Figure 6b, the defined percentage (X%) may be set at 88% and 97% with each of these percentages representing a tail of one of the two peaks in Figure 6b.

In a third step, only a proportion of the references which do not meet the score criteria are retained to reduce the overall size of the genus-level database. Thus, the number of references below the threshold is reduced by keeping just one in a defined number of references (i.e. 1 in Y). For example, the defined number may be 2, 5, 10 or 20 and hence the proportion which is retained may be 50%, 20%, 10% or 5%. The table below shows the number of references which appear in the genus level database using the threshold percentages (88% and 97%) and the defined numbers (2, 5, 10 and 20):

| Threshold percentage (X) | Defined amount (Y) | Database size (Gb) | % of whole bacillus database |
|---|---|---|---|
| 100 (i.e. all) | 1 (i.e. all) | 2.66 | 100 |
| 88 | 2 | 1.82 | 68.29 |

(continued)

| Threshold percentage (X) | Defined amount (Y) | Database size (Gb) | % of whole bacillus database |
|---|---|---|---|
| 88 | 5 | 1.31 | 49.15 |
| 88 | 10 | 1.14 | 42.83 |
| 88 | 20 | 1.06 | 39.74 |
| 97 | 2 | 1.52 | 57.20 |
| 97 | 5 | 0.84 | 31.73 |
| 97 | 10 | 0.61 | 23.12 |
| 97 | 20 | 0.50 | 18.62 |

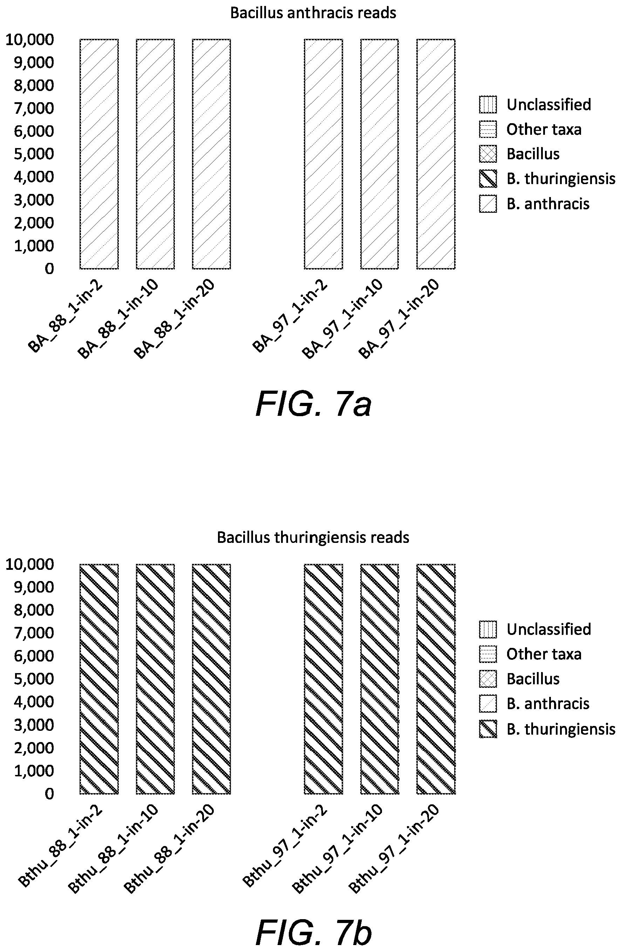

Figure 7a shows the results of using the reduced size genus level databases (1 in 2, 1 in 10 and 1 in 20) shown in the table above when classifying bacillus anthracis. As shown 100% of the 10,000 reads were correctly classified as bacillus anthracis for each of the databases except for the 88% threshold percentage and defined amount of 5 (i.e. the BA_88_1_in_5 database). Figure 7b shows the results of classifying the non-pathogenic bacillus thuringiensis bacterium using the same bacillus database for Figure 7a.

The table below shows relative speed indications using the reduced size genus level database. The times are merely illustrative because it is expected that the final hardware on which the process is carried out which have a higher specification than the test equipment. Nevertheless, the table below shows that the 97%, 1 in 20 database provides identical performance (i.e. all reads correctly identified for both bacillus anthracis and bacillus thuringiensis) but only takes 20% of the time to process the results:

| Database | Mean CPU time | Mean % CPU time | Mean elapsed time | Mean % elapsed time |
|---|---|---|---|---|
| Complete | 04:40:16 | 100.00 | 01:10:04 | 100.00 |
| 88_1-in-2 | 02:23:20 | 51.14 | 00:35:50 | 51.14 |
| 88_1-in-10 | 02:11:46 | 47.01 | 00:32:57 | 47.01 |
| 88_1-in-20 | 02:01:18 | 43.28 | 00:30:19 | 43.28 |
| 97_1-in-2 | 02:00:00 | 42.82 | 00:30:00 | 42.82 |
| 97_1-in-5 | 01:11:24 | 25.48 | 00:17:51 | 25.48 |
| 97_1-in-10 | 01:01:14 | 21.85 | 00:15:18 | 21.85 |
| 97_1-in-20 | 00:53:36 | 19.12 | 00:13:24 | 19.12 |

Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification.

All of the features disclosed in this specification (including any accompanying claims, abstract and drawings), and/or all of the steps of any method or process so disclosed, may be combined in any combination, except combinations where at least some of such features and/or steps are mutually exclusive. Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiments.

List of references cited

"Optimized DNA extraction and metagenomic sequencing of airborne microbial communities" by Jiang et al, published in Nature Protocols 10, 768-799 (2015)

"Longitudinal survey of microbiome associated with particular matter in a megacity" by Qin et al published in Genome Biology 21, Article number: 55 (2020)

"Characterization of the public transit air microbiome and resistome reveals geographical specificity" by Leung et al published in Microbiome 9, Article number: 112 (2021)

"Basic Local Alignment Search Tool" by Altschul et al published in Journal of Molecule Biology 1009, Oct 5; 215(3):403-10

"Improved metagenomic analysis with Kraken 2" by Wood et al in Genome Biol, 2019; 20(1): 257

"BLAST: at the core of a powerful and diverse set of sequence analysis tools" by McGinnis et al published in Nucleic Acids Research, Volume 32, Issue suppl_2, 1 July 2004, Pages W20-W25

"MetaMaps - Strain-level metagenomic assignment and compositional estimation for long reads" by Dilthey et al published in Nature Communications doi: 10.1038/s41467-019-10934-2

"Accurate read-based metagenome characterisation using a hierarchical suite of unique signatures" by Freitas et al published in Nucleic Acids Research, Volume 43, Issue 10, 26 May 2015

"VFDB2019: a comparative pathogenomic platform with an interactive web interface" by Liu et al published in Nucleic Acids Res. 47 (D1): D687-D692

**Claims**

1. A real-time computer-implemented method for detecting a pathogen in a sample, the method comprising:

    inputting a plurality of sequence reads which are obtained from the sample using nucleic acid sequencing; and

    for each input sequence read in the plurality of sequence reads:

        comparing, using a first classification algorithm, the input sequence read to a plurality of target sequences in a first database, wherein the first database comprises nucleic acid sequences for pathogens;
        identifying, using the first classification algorithm, a set of hits for the input sequence read, wherein each hit is a target sequence which matches the input sequence read;
        assigning, using the set of hits, the input sequence read to a taxon; and
        classifying the input sequence read as potentially pathogenic when the taxon to which the input sequence read is assigned corresponds to a pathogen taxon; and

    when the input sequence read is classified as potentially pathogenic:

        comparing, using a second classification algorithm which is different to the first classification algorithm, the potentially pathogenic input sequence read to a plurality of target sequences in a second database, wherein the second database is selected based on the pathogen taxon which corresponds to the taxon to which the input sequence read is assigned; and
        confirming the classification of the potentially pathogenic input sequence read as pathogenic when the potentially pathogenic input sequence read matches one or more target sequences in the second database;

    wherein the method further comprises
    calculating a confidence score for each input sequence read by incrementing the confidence score by a first fixed amount when the input sequence read is classified as potentially pathogenic and by incrementing the confidence score by a second fixed amount when each input sequence read which is classified as potentially pathogenic is confirmed to be pathogenic;
    comparing the confidence score to a confidence score threshold to determine whether each input sequence read is a match to a pathogen; and
    for each pathogen which is identified as a match to an input sequence read:

        determining the number of input sequence reads $n_p$ which are determined to be a match to the pathogen;
        calculating a pathogen confidence score associated with the pathogen from each confidence score for an input sequence read for the pathogen;

dividing a genome for the identified pathogen into a plurality of segments;

counting the number of input sequence reads in each segment which are determined to be a match to the pathogen,

calculating, using the counted number of input sequence reads in each segment, a genome spread value which is indicative of a distribution of the pathogenic input sequence reads across the genome for the identified pathogen; and

generating an overall score for the pathogen using a weighted sum of at least the pathogen confidence score and the genome spread value.

2. The method of claim 1, wherein the first classification algorithm is selected from a seed and extend local alignment search tool and a k-mer matching tool and wherein the second classification algorithm is a strain-level metagenomic assignment and compositional estimation algorithm, MetaMaps.

3. The method of any one of the preceding claims, wherein assigning the input sequence read to a taxon comprises:

comparing taxonomic paths for each hit within the set of hits, and
selecting the lowest common ancestor within each compared taxonomic path as the taxon.

4. The method of any one of the preceding claims, wherein classifying the input sequence read as potentially pathogenic further comprises determining whether additional criteria are met, and.
wherein the additional criteria include one or more of an identity value exceeding an identity threshold, a query cover value exceeding a query cover threshold, and the input sequence read having a minimum length.

5. The method of any one of the preceding claims, further comprising

determining whether the input sequence read is associated with a virulence factor and
incrementing the confidence score by a third fixed amount when the input sequence is determined to be virulent
wherein the third fixed amount is smaller than the first and second fixed amounts.

6. The method of any one of the preceding claims, wherein the first fixed amount is equal to the second fixed amount.

7. The method of any one of the preceding claims, wherein a pathogen-to-relative ratio $R_p$ is calculated as

$$R_p = \frac{n_p}{m_g\left(n_g - n_p\right) + n_p}$$

where $n_p$ is the number of input sequence reads which determined to be a match to a particular pathogen, $n_g$ is the number of input sequence reads classified to the same genus as the particular pathogen and $m_g$ is a per-genus multiplier and
the overall score is determined using a weighted sum comprising the pathogen-to-relative ratio.

8. The method of any one of the preceding claims,
wherein the genome spread value $g_s$ is calculated as

$$g_s = 1 - V_d$$

where $V_d$ is a total variation distance which is calculated by comparing a distribution of the counted number of input sequence reads in each segment to a uniform distribution across the pathogen of input sequence reads which have been classified as pathogenic.

9. The method of any one of the preceding claims, further comprising

determining the number of input sequence reads $n_u$ which are determined to be a match to a particular pathogen and which are unique to that particular pathogen;
determining the number of input sequence reads $n_n$ which determined to be a match to the particular pathogen and which are not unique to that particular pathogen;
calculating a uniqueness ratio from:

$$U_p = \frac{n_u}{n_n}$$

where $n_u$ is the number of unique pathogenic reads and $n_n$ is the number of non-unique pathogenic reads; and
including the uniqueness ratio in the overall score.

10. The method of any one of the preceding claims, further comprising, for each input sequence read which is determined to be a match to a particular pathogen:

   determining whether the input sequence read is unique, and
   when the input sequence read is unique, incrementing the confidence score by a fourth fixed amount.

11. The method of any one of the preceding claims, further comprising

   determining the number of input sequence reads $n_u$ which are determined to be a match to a particular pathogen and which are unique to that particular pathogen;
   determining the number of input sequence reads $n_n$ which determined to be a match to the particular pathogen and which are not unique to that particular pathogen;
   calculating a pathogen to relative ratio using the number of input sequence reads $n_p$ which are a match to a particular pathogen and the number of input sequence reads $n_g$ which are a match to the genus of the particular pathogen;
   calculating a uniqueness ratio using the number of unique pathogenic reads $n_u$ and the number of non-unique pathogenic reads $n_n$, and
   outputting an overall score $S_P$ for the particular pathogen using a weighted sum of the pathogen confidence score, the pathogen to relative ratio, the genome spread value and the uniqueness ratio, wherein the weighted sum is defined as

$$S_P = n_p\, s_{mean}\, (1 + \alpha R_p + \beta s_g + \delta U_p)$$

   where $n_p$ is the number of input sequence reads which are determined to be a match to a particular pathogen, $s_{mean}$ is the pathogen confidence score, $R_p$ Is the pathogen-to-relative ratio, $s_g$ is the genome spread value, $U_p$ is the uniqueness ratio and $\alpha, \beta, \delta$ are constants.

12. The method of any one of the preceding claims, further comprising obtaining an overall score for the sample from

$$S_S = \sum_{i=1}^{q} S_p$$

   where $S_P$ is the score for a particular pathogen, q is the number of pathogens identified in the sample and $S_S$ is the overall score for the sample.

13. The method of any one of the preceding claims wherein the pathogen being detected is a bacterial, fungal or viral pathogen and/or wherein the sample is an environmental sample.

14. A non-transitory data carrier carrying processor control code, which when executed by a processor, causes the processor to carry out the method of any of the preceding claims.

15. A system comprising one or more processors and memory, the one or more processors being configured to execute instructions in said memory to cause the system to carry out the method of any one of claims 1 to 13.

**Patentansprüche**

1. Computerimplementiertes Echtzeit-Verfahren zum Nachweisen eines Pathogens in einer Probe, das Verfahren Folgendes umfassend:

   Eingeben einer Vielzahl von Sequenzlesungen, die aus der Probe unter Verwendung von Nukleinsäuresequenzieren erhalten wurden; und
   für jede eingegebene Sequenz, die in der Vielzahl von Sequenzlesungen gelesen wurde:

   Vergleichen, unter Verwendung eines ersten Klassifizierungsalgorithmus, der eingegebenen Sequenzlesung mit einer Vielzahl von Zielsequenzen in einer ersten Datenbank, wobei die erste Datenbank Nukleinsäuresequenzen für Pathogene umfasst;
   Identifizieren, unter Verwendung des ersten Klassifizierungsalgorithmus, eines Satzes von Treffern für die eingegebene Sequenzlesung, wobei jeder Treffer eine Zielsequenz ist, die mit der eingegebenen Sequenzlesung übereinstimmt;
   Zuweisen, unter Verwendung des Satzes von Treffern, der eingegebenen Sequenzlesung zu einem Taxon; und
   Klassifizieren der eingegebenen Sequenzlesung als potenziell pathogen, wenn das Taxon, dem die eingegebene Sequenzlesung zugewiesen ist, einem Pathogen-Taxon entspricht; und
   wenn die eingegebene Sequenzlesung als potenziell pathogen klassifiziert wird:

   Vergleichen, unter Verwendung eines zweiten Klassifizierungsalgorithmus, der sich von dem ersten Klassifizierungsalgorithmus unterscheidet, der potenziell pathogenen eingegebenen Sequenzlesung mit einer Vielzahl von Zielsequenzen in einer zweiten Datenbank, wobei die zweite Datenbank basierend auf dem Pathogen-Taxon ausgewählt wird, das dem Taxon entspricht, dem die eingegebene Sequenzlesung zugewiesen ist; und
   Bestätigen der Klassifizierung der potenziell pathogenen eingegebenen Sequenzlesung als pathogen, wenn die potentiell pathogene eingegebene Sequenzlesung mit einer oder mehreren Zielsequenzen in der zweiten Datenbank übereinstimmt;
   wobei das Verfahren ferner umfasst
   Berechnen einer Konfidenzbewertungsziffer für jede eingegebene Sequenzlesung durch Inkrementieren der Konfidenzbewertungsziffer um einen ersten festen Betrag, wenn die eingegebene Sequenzlesung als potenziell pathogen klassifiziert wird, und durch Inkrementieren der Konfidenzbewertungsziffer um einen zweiten festen Betrag, wenn jede eingegebene Sequenzlesung, die als potenziell pathogen klassifiziert ist, als pathogen bestätigt wird;
   Vergleichen der Konfidenzbewertungsziffer mit einem Konfidenzbewertungsschwellenwert, um zu bestimmen, ob jede eingegebene Sequenzlesung eine Übereinstimmung mit einem Pathogen ist; und
   für jedes Pathogen, das als Übereinstimmung mit einer eingegebenen Sequenzlesung identifiziert wird:

   Bestimmen der Anzahl von eingegebenen Sequenzlesungen $n_p$, die als eine Übereinstimmung mit dem Pathogen bestimmt werden;
   Berechnen einer Pathogen-Konfidenzbewertungsziffer, die mit dem Pathogen assoziiert ist, aus jeder Konfidenzbewertungsziffer für eine eingegebene Sequenzlesung für das Pathogen;
   Aufteilen eines Genoms für das identifizierte Pathogen in eine Vielzahl von Segmenten;
   Zählen der Anzahl von eingegebenen Sequenzlesungen in jedem Segment, von denen bestimmt wird, dass sie mit dem Pathogen übereinstimmen,
   Berechnen, unter Verwendung der gezählten Anzahl von eingegebenen Sequenzlesungen in jedem Segment, eines Genomstreuwerts, der eine Verteilung der pathogenen eingegebenen Sequenzlesung über das Genom für das identifizierte Pathogen angibt; und
   Erzeugen einer Gesamtbewertungsziffer für das Pathogen unter Verwendung einer gewichteten Summe aus mindestens der Pathogen-Konfidenzbewertungsziffer und dem Genomstreuwert.

2. Verfahren nach Anspruch 1, wobei der erste Klassifizierungsalgorithmus aus einem Seed-and-Extend-Local-Alignment-Suchwerkzeug und einem k-mer-Matching-Werkzeug ausgewählt ist und wobei der zweite Klassifizierungsalgorithmus ein Metagenom-Zuweisungs- und Kompositionsschätzalgorithmus auf Stammebene, MetaMaps, ist.

3. Verfahren nach einem der vorhergehenden Ansprüche, wobei Zuweisen der eingegebenen Sequenzlesung zu einem Taxon Folgendes umfasst:

Vergleichen von taxonomischen Pfaden für jeden Treffer innerhalb des Satzes von Treffern und
Auswählen des untersten gemeinsamen Vorfahrens in jedem verglichenen taxonomischen Pfad als das Taxon.

4. Verfahren nach einem der vorhergehenden Ansprüche, wobei Klassifizieren der eingegebenen Sequenzlesung als potenziell pathogen ferner Bestimmen umfasst, ob zusätzliche Kriterien erfüllt sind, und
wobei die zusätzlichen Kriterien eines oder mehreres von einem Identitätswert, der einen Identitätsschwellenwert überschreitet, einem Abfragedeckungswert, der einen Abfragedeckungsschwellenwert überschreitet, und der eingegebenen Sequenzlesung mit einer Mindestlänge umfassen.

5. Verfahren nach einem der vorhergehenden Ansprüche, ferner Folgendes umfassend

Bestimmen, ob die eingegebene Sequenzlesung mit einem Virulenzfaktor assoziiert ist, und
Inkrementieren der Konfidenzbewertungsziffer um einen dritten festen Betrag, wenn bestimmt wird, dass die eingegebene Sequenz virulent ist,
wobei der dritte feste Betrag kleiner ist als der erste und der zweite feste Betrag.

6. Verfahren nach einem der vorhergehenden Ansprüche, wobei der erste feste Betrag gleich dem zweiten festen Betrag ist.

7. Verfahren nach einem der vorhergehenden Ansprüche, wobei ein Verhältnis von Pathogen zu Verwandten $R_p$ berechnet wird als

$$R_p = \frac{n_p}{m_g(n_g - n_p) + n_p}$$

wobei $n_p$ die Anzahl von eingegebenen Sequenzlesungen ist, die als eine Übereinstimmung mit einem bestimmten Pathogen bestimmt wurden, $n_g$ die Anzahl von eingegebenen Sequenzlesungen ist, die in dieselbe Gattung wie das bestimmte Pathogen klassifiziert sind, und $m_g$ ein Multiplikator pro Gattung ist und
die Gesamtbewertungsziffer unter Verwendung einer gewichteten Summe aus dem Verhältnis von Pathogen zu Verwandten bestimmt wird.

8. Verfahren nach einem der vorhergehenden Ansprüche, wobei der Genomstreuwert $g_s$ berechnet wird als

$$g_s = 1 - V_d$$

wobei $V_d$ ein Gesamtvariationsabstand ist, der durch Vergleichen einer Verteilung der gezählten Anzahl von eingegebenen Sequenzlesungen in jedem Segment mit einer einheitlichen Verteilung von eingegebenen Sequenzlesungen, die als pathogen klassifiziert wurden, über das Pathogen hinweg berechnet wird.

9. Verfahren nach einem der vorhergehenden Ansprüche, ferner Folgendes umfassend

Bestimmen der Anzahl von eingegebenen Sequenzlesungen $n_u$, die als mit einem bestimmten Pathogen übereinstimmend bestimmt wurden und die für dieses bestimmte Pathogen eindeutig sind;
Bestimmen der Anzahl der eingegebenen Sequenzlesungen $n_n$, die als mit dem bestimmten Pathogen übereinstimmend bestimmt wurden und die für dieses bestimmte Pathogen nicht eindeutig sind;
Berechnen eines Eindeutigkeitsverhältnisses aus:

$$U_p = \frac{n_u}{n_n}$$

wobei $n_u$ die Anzahl der eindeutigen pathogenen Lesungen und $n_n$ die Anzahl der nicht eindeutigen pathogenen Lesungen ist; und
Einbeziehen des Eindeutigkeitsverhältnisses in die Gesamtbewertungsziffer.

10. Verfahren nach einem der vorhergehenden Ansprüche, das ferner für jede eingegebene Sequenzlesung, die als eine Übereinstimmung mit einem bestimmten Pathogen bestimmt wird, Folgendes umfasst:

Bestimmen, ob die eingegebene Sequenzlesung eindeutig ist, und,
wenn die eingegebene Sequenzlesung eindeutig ist, Inkrementieren der Konfidenzbewertungsziffer um einen vierten festen Betrag.

**11.** Verfahren nach einem der vorhergehenden Ansprüche, ferner Folgendes umfassend

Bestimmen der Anzahl von eingegebenen Sequenzlesungen $n_u$, die als mit einem bestimmten Pathogen übereinstimmend bestimmt wurden und die für dieses bestimmte Pathogen eindeutig sind;
Bestimmen der Anzahl der eingegebenen Sequenzlesungen $n_n$, die als mit dem bestimmten Pathogen übereinstimmend bestimmt wurden und die für dieses bestimmte Pathogen nicht eindeutig sind;
Berechnen eines Verhältnisses von Pathogen zu Verwandten unter Verwendung der Anzahl eingegebener Sequenzlesungen $n_p$, die mit einem bestimmten Pathogen übereinstimmen, und der Anzahl eingegebener Sequenzlesungen $n_g$, die mit der Gattung des bestimmten Pathogens übereinstimmen;
Berechnen eines Eindeutigkeitsverhältnisses unter Verwendung der Anzahl der eindeutigen pathogenen Lesungen $n_u$ und der Anzahl der nicht eindeutigen pathogenen Lesungen $n_n$ und
Ausgeben einer Gesamtbewertungsziffer $S_P$ für das bestimmte Pathogen unter Verwendung einer gewichteten Summe der Pathogen-Konfidenzbewertungsziffer, des Verhältnisses von Pathogen zu Verwandten, des Genomstreuwerts und des Eindeutigkeitsverhältnisses, wobei die gewichtete Summe definiert ist als

$$S_P = n_p s_{mean} \ (1 \ + \ \alpha R_p \ + \ \beta s_g \ + \ \delta U_p)$$

wobei $n_p$ die Anzahl der eingegebenen Sequenzlesungen ist, die als eine Übereinstimmung mit einem bestimmten Pathogen bestimmt werden, $s_{mean}$ die Pathogen-Konfidenzbewertungsziffer ist, $R_p$ das Verhältnis von Pathogen zu Verwandten ist, $s_g$ der Genomstreuwert ist, $U_p$ das Eindeutigkeitsverhältnis ist und $\alpha$, $\beta$, $\delta$ Konstanten sind.

**12.** Verfahren nach einem der vorhergehenden Ansprüche, ferner umfassend Erhalten einer Gesamtbewertungsziffer für die Probe aus

$$S_S = \sum_{i=1}^{q} S_p$$

wobei $S_P$ die Bewertungsziffer für ein bestimmtes Pathogen ist, q die Anzahl der in der Probe identifizierten Pathogene ist und $S_S$ die Gesamtbewertungsziffer für die Probe ist.

**13.** Verfahren nach einem der vorhergehenden Ansprüche, wobei das Pathogen, das nachgewiesen wird, ein bakterielles, pilzartiges oder virales Pathogen ist und/oder wobei die Probe eine Umweltprobe ist.

**14.** Nichtflüchtiger Datenträger, der einen Prozessorsteuerungscode trägt, der, wenn er von einem Prozessor ausgeführt wird, den Prozessor veranlasst, das Verfahren nach einem der vorhergehenden Ansprüche auszuführen.

**15.** System, das einen oder mehrere Prozessoren und Speicher umfasst, wobei der eine oder die mehreren Prozessoren dazu ausgelegt sind, Anweisungen in dem Speicher auszuführen, um das System zu veranlassen, das Verfahren nach einem der Ansprüche 1 bis 13 auszuführen.

**Revendications**

**1.** Procédé, mis en œuvre par ordinateur, en temps réel, pour la détection d'un agent pathogène dans un échantillon, le procédé comprenant :

la saisie d'une pluralité de lectures de séquences qui sont obtenues à partir de l'échantillon au moyen du séquençage d'acide nucléique ; et
pour chaque lecture de séquence d'entrée dans la pluralité de lectures de séquences :

la comparaison, au moyen d'un premier algorithme de classification, de la lecture de séquence d'entrée avec une pluralité de séquences cibles dans une première base de données, la première base de données comprenant des séquences d'acides nucléiques de pathogènes ;

l'identification, au moyen du premier algorithme de classification, d'un ensemble de correspondances pour la lecture de séquence d'entrée, chaque correspondance étant une séquence cible qui correspond à la lecture de séquence d'entrée ;

l'affectation, au moyen de l'ensemble de correspondances, de la lecture de séquence d'entrée à un taxon ; et la classification de la lecture de séquence d'entrée comme potentiellement pathogène lorsque le taxon auquel la lecture de séquence d'entrée est affectée correspond à un taxon pathogène ; et lorsque la lecture de séquence d'entrée est classée comme potentiellement pathogène :

la comparaison, au moyen d'un second algorithme de classification différent du premier algorithme de classification, de la lecture de séquence d'entrée potentiellement pathogène avec une pluralité de séquences cibles dans une seconde base de données, la seconde base de données étant sélectionnée sur la base du taxon pathogène qui correspond au taxon auquel la lecture de séquence d'entrée est affectée ; et

la confirmation de la classification de la lecture de séquence d'entrée potentiellement pathogène comme pathogène lorsque la lecture de séquence d'entrée potentiellement pathogène correspond à une ou plusieurs séquences cibles dans la seconde base de données ;

le procédé comprenant en outre

le calcul d'un score de confiance pour chaque lecture de séquence d'entrée par incrémentation du score de confiance par une première quantité fixe lorsque la lecture de séquence d'entrée est classée comme potentiellement pathogène et par incrémentation du score de confiance par une deuxième quantité fixe lorsque chaque lecture de séquence d'entrée classée comme potentiellement pathogène est confirmée comme étant pathogène ;

la comparaison du score de confiance avec un seuil de score de confiance pour déterminer si chaque lecture de séquence d'entrée correspond à un agent pathogène ; et

pour chaque agent pathogène identifié comme correspondant à une lecture de séquence d'entrée :

la détermination du nombre de lectures de séquences d'entrée $n_p$ qui sont déterminées comme correspondant à l'agent pathogène ;

le calcul d'un score de confiance de pathogène associé à l'agent pathogène à partir de chaque score de confiance pour une lecture de séquence d'entrée pour l'agent pathogène ;

la division d'un génome pour l'agent pathogène identifié en une pluralité de segments ;

le comptage du nombre de lectures de séquences d'entrée dans chaque segment qui sont déterminées comme correspondant à l'agent pathogène ;

le calcul, au moyen du nombre compté de lectures de séquences d'entrée dans chaque segment, d'une valeur de répartition du génome qui est indicative d'une distribution des lectures de séquences d'entrée pathogènes à travers le génome pour l'agent pathogène identifié ; et

la génération d'un score global pour l'agent pathogène au moyen d'une somme pondérée comprenant au moins le score de confiance du pathogène et la valeur de répartition du génome.

2. Procédé selon la revendication 1, le premier algorithme de classification étant sélectionné parmi un outil de recherche d'alignement local par amorce et extension et un outil de mise en correspondance par k-mer et le second algorithme de classification étant un algorithme d'affectation métagénomique au niveau de la souche et d'estimation de composition, MetaMaps.

3. Procédé selon l'une quelconque des revendications précédentes, l'affectation de la lecture de séquence d'entrée à un taxon comprenant :

la comparaison des chemins taxonomiques pour chaque correspondance au sein de l'ensemble de correspondances ; et

la sélection du plus bas ancêtre commun au sein de chaque chemin taxonomique comparé comme taxon.

4. Procédé selon l'une quelconque des revendications précédentes, la classification de la lecture de séquence d'entrée comme potentiellement pathogène comprenant en outre la détermination de la satisfaction de critères supplémentaires, et

les critères supplémentaires comprenant un ou plusieurs éléments parmi une valeur d'identité dépassant un seuil d'identité, une valeur de couverture de requête dépassant un seuil de couverture de requête, et la lecture de séquence d'entrée ayant une longueur minimale.

**5.** Procédé selon l'une quelconque des revendications précédentes, comprenant en outre

la détermination du fait que la lecture de séquence d'entrée est associée à un facteur de virulence ; et l'incrémentation du score de confiance par une troisième quantité fixe lorsque la séquence d'entrée est déterminée comme étant virulente ; la troisième quantité fixe étant inférieure aux première et deuxième quantités fixes.

**6.** Procédé selon l'une quelconque des revendications précédentes, la première quantité fixe étant égale à la deuxième quantité fixe.

**7.** Procédé selon l'une quelconque des revendications précédentes, un rapport pathogène/relatif $R_p$ étant calculé comme

$$R_p = \frac{n_p}{m_g(n_g - n_p) + n_p}$$

$n_p$ étant le nombre de lectures de séquences d'entrée qui sont déterminées comme correspondant à un agent pathogène particulier, $n_g$ étant le nombre de lectures de séquences d'entrée classées dans le même genre que l'agent pathogène particulier et $m_g$ étant un multiplicateur par genre ; et le score global étant déterminé au moyen d'une somme pondérée comprenant le rapport pathogène/relatif.

**8.** Procédé selon l'une quelconque des revendications précédentes, la valeur de répartition du génome $g_s$ étant calculée comme

$$g_s = 1 - V_d$$

$V_d$ étant une distance de variation totale qui est calculée en comparant une distribution du nombre compté de lectures de séquences d'entrée dans chaque segment à une distribution uniforme à travers l'agent pathogène de lectures de séquences d'entrée qui ont été classées comme pathogènes.

**9.** Procédé selon l'une quelconque des revendications précédentes, comprenant en outre

la détermination du nombre de lectures de séquences d'entrée $n_u$ qui sont déterminées comme correspondant à un agent pathogène particulier et qui sont uniques à cet agent pathogène particulier ; la détermination du nombre de lectures de séquences d'entrée $n_n$ qui sont déterminées comme correspondant à l'agent pathogène particulier et qui ne sont pas uniques à cet agent pathogène particulier ; le calcul d'un rapport d'unicité à partir de :

$$U_p = \frac{n_u}{n_n}$$

$n_u$ étant le nombre de lectures pathogènes uniques et $n_n$ étant le nombre de lectures pathogènes non uniques ; et l'inclusion du rapport d'unicité dans le score global.

**10.** Procédé selon l'une quelconque des revendications précédentes, comprenant en outre, pour chaque lecture de séquence d'entrée qui est déterminée comme correspondant à un agent pathogène particulier :

la détermination du fait que la lecture de séquence d'entrée est unique ; et lorsque la lecture de séquence d'entrée est unique, l'incrémentation du score de confiance par une quatrième quantité fixe.

**11.** Procédé selon l'une quelconque des revendications précédentes, comprenant en outre

la détermination du nombre de lectures de séquences d'entrée $n_u$ qui sont déterminées comme correspondant à un agent pathogène particulier et qui sont uniques à cet agent pathogène particulier ;

la détermination du nombre de lectures de séquences d'entrée $n_n$ qui sont déterminées comme correspondant à l'agent pathogène particulier et qui ne sont pas uniques à cet agent pathogène particulier ;

le calcul d'un rapport pathogène/relatif en utilisant le nombre de lectures de séquences d'entrée $n_p$ qui correspondent à un agent pathogène particulier et le nombre de lectures de séquences d'entrée $n_g$ qui correspondent au genre du pathogène particulier ;

le calcul d'un rapport d'unicité en utilisant le nombre de lectures pathogènes uniques $n_u$ et le nombre de lectures pathogènes non uniques $n_n$ ; et

la production d'un score global $S_P$ pour l'agent pathogène particulier au moyen d'une somme pondérée du score de confiance du pathogène, du rapport pathogène/relatif, de la valeur de répartition du génome et du rapport d'unicité, la somme pondérée étant définie comme

$$S_P = n_p s_{mean}\ (1\ +\ \alpha R_p\ +\ \beta s_g\ +\ \delta U_p)$$

$n_p$ étant le nombre de lectures de séquences d'entrée qui sont déterminées comme correspondant à un agent pathogène particulier, $s_{mean}$ étant le score de confiance du pathogène, $R_p$ étant le rapport pathogène/relatif, $s_g$ étant la valeur de répartition du génome, $U_p$ étant le rapport d'unicité et $\alpha$, $\beta$, $\delta$ étant des constantes.

12. Procédé selon l'une quelconque des revendications précédentes, comprenant en outre l'obtention d'un score global pour l'échantillon à partir de

$$S_S = \sum_{i=1}^{q} S_p$$

$S_P$ étant le score pour un agent pathogène particulier, q étant le nombre d'agents pathogènes identifiés dans l'échantillon et $S_S$ étant le score global de l'échantillon.

13. Procédé selon l'une quelconque des revendications précédentes, l'agent pathogène détecté étant un agent pathogène bactérien, fongique ou viral et/ou l'échantillon étant un échantillon environnemental.

14. Support de données non transitoire portant un code de commande de processeur, qui, lorsqu'il est exécuté par un processeur, amène le processeur à réaliser le procédé selon l'une quelconque des revendications précédentes.

15. Système comprenant un ou plusieurs processeurs et une mémoire, le ou les processeurs étant configurés pour exécuter des instructions dans ladite mémoire pour amener le système à réaliser le procédé selon l'une quelconque des revendications 1 à 13.

Input a sequence read
S100

(Optional) Filter to remove read if low quality
S102

**Classification phase**

Compare sequence read to a bacteria and virus database
S106

Hit(s) identified?
S108

Compare taxonomic paths for hit(s) S110

Pathogen hit confirmed? S112

yes

Update confidence score
S114

**Confirmation phase**

Compare classified read to a pathogen database
S116

Pathogen?
S118

yes

Update confidence score
S120

Compare sequence read(s) to a virulence factor database
S206

Match?
S208

yes

Update confidence score
S214

Determine whether there is a genuine match based on confidence score S216

Pathogen?
S218

yes

Output alert
S220

*FIG. 1*

Escherichia coli O25

Escherichia coli PCN061

Klebsiella pneumoniae 500_1420

Klebsiella pneumoniae subsp. pneumoniae

Escherichia coli

Klebsiella pneumoniae

Enterobacteriaceae

*FIG. 2*

FIG. 3a

Input a plurality of sequence reads
from a sample
S300

Classify each of the plurality of sequence reads
as pathogenic or non-pathogenic
S302

no ← Pathogen
identified? S304

yes

Determine the number of reads classified as
pathogenic for the given pathogen
S306

Determine the number of reads classified as
pathogenic for pathogens in same genus
S408

(Optional) Obtain a per-genus multiplier
S410

Calculate pathogen-to-relative ratio
S412

Ratio above
threshold? S414 → no

yes

Output alert
S416

*FIG. 3b*

Input a plurality of sequence reads
from a sample
S300

Classify each of the plurality of sequence reads
as pathogenic or non-pathogenic
S302

Pathogen
identified? S304

no

yes

Divide the genome for the identified pathogen
into a plurality of segments
S506

Count how many reads which are identified as
pathogenic are in each segment
S508

Calculate the mean number of reads per
segment for the pathogen S510

Determine a genome spread value from the
mean number and counted number of reads
S512

Spread below
threshold? S514

no

yes

Output alert
S516

## FIG. 3c

Input a plurality of sequence reads
from a sample
S300

Classify each of the plurality of sequence reads
as pathogenic or non-pathogenic
S302

Pathogen
identified? S304

no

yes

Determine the number of
unique pathogenic reads S606

Determine the number of
non-unique pathogenic reads S608

Calculate the ratio of unique to
non-unique reads S610

Ratio above
threshold? S612

no

yes

Output alert
S614

*FIG. 3d*

*FIG. 3e*

FIG. 4a

FIG. 4b

EP 4 460 830 B1

FIG. 5

36

*FIG. 6a*

FIG. 6b

FIG. 7a

FIG. 7b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2021222231 A

- US 2018365375 A1

### Non-patent literature cited in the description

- **JIANG et al.** Optimized DNA extraction and metagenomic sequencing of airborne microbial communities. *Nature Protocols*, 2015, vol. 10, 768-799
- **ALTSCHUL et al.** Basic Local Alignment Search Tool. *Journal of Molecule Biology 1009*, vol. 215 (3), 403-10
- **WOOD et al.** Improved metagenomic analysis with Kraken 2. *Genome Biol*, 2019, vol. 20 (1), 257
- **O'LEARY et al.** Reference sequence (RefSeq) database at NCBI: current status, taxonomic expansion and functional annotation. *Nucleic Acids Res*, 04 January 2016, vol. 44 (D1), 733-45
- **BOWERS et al.** Microbial Ecology. 2011
- **MCGINNIS et al.** BLAST: at the core of a powerful and diverse set of sequence analysis tools. *Nucleic Acids Research*, 01 July 2004, vol. 32, W20-W25
- **DILTHEY et al.** MetaMaps - Strain-level metagenomic assignment and compositional estimation for long reads. *Nature Communications*

- **FREITAS et al.** Accurate read-based metagenome characterisation using a hierarchical suite of unique signatures. *Nucleic Acids Research*, 26 May 2015, vol. 43 (10)
- **PRITCHARD et al.** Genomics and taxonomy in diagnostics for food security: soft-rotting enterobacterial plant pathogens. *Journal of the Royal Society of Chemistry*, 2015
- **QIN et al.** Longitudinal survey of microbiome associated with particular matter in a megacity. *Genome Biology*, 2020, vol. 21 (55)
- **LEUNG et al.** Characterization of the public transit air microbiome and resistome reveals geographical specificity. *Microbiome*, 2021, vol. 9 (112)
- **ALTSCHUL et al.** Basic Local Alignment Search Tool. *Journal of Molecule Biology 1009, Oct 5*, vol. 215 (3), 403-10
- **WOOD** ; **2019 et al.** Improved metagenomic analysis with Kraken 2. *Genome Biol*, vol. 20 (1), 257
- **LIU et al.** VFDB2019: a comparative pathogenomic platform with an interactive web interface. *Nucleic Acids Res.*, vol. 47 (D1), D687-D692